# EUROPEAN PATENT SPECIFICATION

(11) **EP 1 917 226 B1**
(45) Date of publication and mention of the grant of the patent: **10.06.2009**
(21) Application number: 06801905.8
(22) Date of filing: 21.08.2006
(51) Int. Cl.: C07C 7/144, C07C 6/04, C07C 11/08, B01J 19/24, B01J 8/00, C07C 5/25

(54) **BUTANE REMOVAL IN C4 UPGRADING PROCESSES**
BUTANENTFERNUNG IN C4-UPGRADING-PROZESSEN
ELIMINATION DE BUTANE DANS LES OPERATIONS DE VALORISATION DE FLUX DE C4

(30) Priority: 23.08.2005 US 209316
(43) Date of publication of application: 07.05.2008
(73) Proprietor: Lummus Technology Inc., Bloomfield NJ 07003-3096 (US)
(72) Inventor: GARTSIDE, Robert, J., Summit, NJ 07901 (US); GREENE, Marvin, I., Hackensack, NJ 07601 (US); JONES, Quincy, J., Manvel TX 77578 (US)
(74) Representative: McKelvey, Ian Edward
(86) International application number: PCT/US2006/032436
(87) International publication number: WO 2007/024733

(56) References cited:
- WO-A-02/096843
- US-A1- 2001 013 273
- US-A1- 2003 052 056
- US-A1- 2005 014 981
- US-B2- 6 727 396
- VAN DE GRAAF J M ET AL: "Application of a silicalite-1 membrane reactor in metathesis reactions" APPLIED CATALYSIS A: GENERAL, ELSEVIER SCIENCE, AMSTERDAM, NL, vol. 178, no. 2, 22 March 1999 (1999-03-22), pages 225-241, XP004271681 ISSN: 0926-860X

## Description

### Background of the Invention

The present invention relates to the processing of a C3 to C5 hydrocarbon cut from a cracking process, such as steam or fluid catalytic cracking, for inter-conversion of C4 and C5 olefins to propylene, ethylene, and hexene via metathesis and for the double bond isomerization or skeletal isomerization of olefins including butenes and pentenes.

Double bond isomerization is a process where the position of the double bond in a molecule is shifted without affecting the structure of the molecule. For example, as described in U.S. Patent No. 6,875,901, a mixture of 1-butene and 2-butene is isomerized to produce a stream of high purity 1-butene. This process nominally occurs over a basic metal oxide catalyst.

Skeletal isomerization is a process where the structure of the molecule is changed via rearrangement of R groups. There is both skeletal isomerization of paraffins (for example isobutane to normal butane) and olefins (for example isobutylene to normal butene). This often occurs over acidic catalysts. Both double bond and skeletal isomerization catalysts are sensitive to the same poisons that impact metathesis catalysts.

These isomerization processes are also directed at the upgrading of olefin streams and the paraffin content, as either iso or normal butane, represents a diluent for the reaction. The isomerization reactions are equilibrium limited and thus require, C4 separation and recycle in order to achieve high conversions of the olefins. The paraffins have boiling points close to the olefins of interest and as such are difficult to remove by fractionation prior to recycle. In conventional processing they build up in the feed to the reactor via recycle and thus limit the processing of the olefins in the feed stream.

Metathesis is also a means of upgrading C4 olefin streams by converting those olefins to more valuable lower olefins. Metathesis is a reaction involving the disproportionation of two olefins to produce two other olefins that differ in carbon number. An example is the metathesis of 2-butene with ethylene to form two propylenes. This technology has been extensively described in the literature. The majority of commercial applications of this technology involve the use of a stream of C4 components as at least one of the olefin feedstocks. The C4 feed stream typically contains C4 olefins, including both normal butenes and isobutenes, and C4 paraffins, including both normal and isobutane. Often, more highly unsaturated materials such as butadiene also are included. In addition, the C4 feed stream may contain minor amounts of C3 or C5 components. In some metathesis catalyst systems, there is an isomerization catalyst in combination with the metathesis catalyst to isomerize the 1-butene to 2-butene and thus allow it to react with ethylene.

Commonly assigned U.S. Patent No. 6,727,396 discloses an autometathesis process in which a C4 cut from a steam or other cracking process is used to produce ethylene and hexene. In autometathesis, the metathesis reaction occurs without the use of ethylene. The mixed C4 stream containing olefins is converted to a feed of essentially high purity 1-butene. The high purity 1-butene stream is fed to the autometathesis reactor, which converts it to ethylene and 3-hexene. The 3-hexene is subsequently isomerized to 1-hexene and is purified.

In order to improve the yield of a desired product from a metathesis process, a number of different processes have been used to prepare the metathesis feed stream. In some cases, a C4 feed stream is hydrogenated to reduce the content of dienes and/or acetylenics to low levels, because these material cause rapid coke buildup on both the isomerization and metathesis catalyst and therefore must be removed or at least minimized in order to provide for useful catalyst cycle times. If selective hydrogenation is used, there can be either one or two stages depending upon the concentration of the highly unsaturated components. When a selective hydrogenation process is employed, some of the unsaturated C4 compounds including butadiene and butenes are hydrogenated to form butanes. Butanes formed via hydrogenation or present in the feed are non-reactive under metathesis conditions. They dilute the reaction mixture and limit the extent to which the olefins can be reacted.

As an alternative, butadiene and acetylenics can be removed by extraction. In many cases, there is some residual butadiene or other dienes or acetylenics remaining after extraction. The remaining highly unsaturated compounds are also removed in a selective hydrogenation unit. Depending upon the processing sequence and the metathesis products desired, in addition to saturation of the butadiene and other highly unsaturated compounds, in a second stage of hydrogenation, hydroisomerization is allowed to occur and the 1-butene is hydro-isomerized to 2-butene. This second reaction step can occur either in a fixed bed or in a catalytic distillation column.

For some types of metathesis the removal of isobutylene is necessary or favorable, while for other types of metathesis isobutylene is a favored reactant. For autometathesis to produce propylene as in U.S. Patent No. 6,777,582, isobutylene is desirable. When autometathesis is used to produce ethylene and linear alpha olefins as in U.S. Patent No. 6.727,396, isobutylene must be removed to low levels. When conventional metathesis is used to produce propylene via the reaction of C4 olefins with ethylene, it is desirable but not necessary to remove isobutylene since it has a low reactivity in mixtures where there is an excess of ethylene. There are a number of ways of removing isobutylene, including by the production of MTBE, fractionation, and isobutylene dimerization technology.

Fractionation can be employed to remove isobutylene and isobutane. Isobutylene and isobutane are light components and are removed overhead. The 1-butene has a relative volatility close to that of isobutylene and significant fractionation is required to avoid losing the normal 1-butene with the isobutylene. The isobutane goes overhead with the isobutylene. The tower bottoms contains 1-butene, 2-butene, n-butane, and any C5 compounds. In some cases, a hydroisomerization reaction can either occur in a fixed bed upstream of the fractionation tower, within the fractionation tower (catalytic distillation), or as a combination of the two. Fractionation will remove the isobutane but not the normal butane. Thus while the total paraffin content of the C4 stream is reduced, significant quantities of paraffins (normal butane) remain.

Isobutylene can also be removed from the C4 stream reactively. However in these cases, only isobutylene is removed and both the iso and normal butanes remain in the C4 feed to metathesis. Isobutylene dimerization technology can be used to remove isobutylene. Isobutylene reacts with itself and some normal butenes to form C8 components. These are separated via fractionation from the remaining C4s. The C4 effluent includes 1-butene, 2-butene, n-butane and isobutane.

The production of MTBE removes isobutylene selectively by reaction with methanol. The effluent from the isobutylene removal step contains the normal butenes (1-butene, 2-butene), n-butane and isobutane if not removed simultaneously with the isobutylene. In addition in all the C4 streams, there are trace oxygen-containing products including DME, methanol, TBA, etc and trace sulfur component such as ethyl mercaptan. These must be removed in subsequent steps prior to metathesis to avoid catalyst poisoning. Some of these poisons will permanently deactivate either the isomerization or the metathesis catalyst. In a metathesis process, adsorbent guard beds are used to remove these poisons to very low levels to improve the process activity. In addition to the poisons noted above, nitrogen compounds such as amides or amines or pyrrolidones are significant poisons. Further, other oxygen compounds such as glycols and sulfur compounds such as sulpholanes and sulphoxides will permanently deactivate the catalysts.

U.S. Publication No. 2003/0220530 discloses a process for preparing olefins in which a paraffin/olefin separation unit is employed upstream of an olefin conversion process. This document proposes this type of separation to reduce the volume of feedstock passing through an olefin conversion unit. The olefin conversion unit is a cracking type process where the longer chain C4 to C6 olefins are cracked to form shorter chain propylene and ethylene using a zeolitic type catalyst. The process does not, however, include any isobutylene removal step because isobutylene is a valuable feedstock for the process disclosed therein. Furthermore, the process disclosed in this document does not employ guard beds since the catalyst that is used is not impacted by poisons.

U.S. Publication No. 2003/0225306 discloses a process for preparing olefins in which a paraffin/olefin separation unit is employed downstream from an olefins conversion reactor system and a fractionator. The paraffin/olefin separation is proposed here to reduce the volume of material passing through the reactor and fractionator as it is recycled to the reaction step. This process also does not include any isobutylene or guard bed poison removal steps.

U.S. Publication Nos. 2003/0220530 and 2003/0225306 provide that the paraffin/olefin separation unit is advantageously equipped with distillation columns designed for extractive distillation. Extractive distillation is a process whereby a polar solvent comprising heavy nitrogen or sulfur compounds such as NMP (N-methylpyrrolidone), DMF (dimethylformamide), acetonitrile, furfural, sulpholane, or diethyleneglycol are contacted with the C4 stream in a fractionating tower. The olefins are selectively absorbed by the polar compounds and are removed from the bottom of the tower with the solvent. The paraffins are removed overhead. The bottoms stream containing the solvent and olefins is then sent to a second distillation column where the olefins are stripped from the heavy solvent. In these systems it is unavoidable that a certain fraction of the solvent is carried overhead with the olefins. The presence of these compounds in the olefins stream would result in the poisoning of the downstream catalyst systems for either metathesis or isomerization.

The unit includes at least two distillation columns with the first being used to separate paraffins and olefins and the second being configured as a regeneration column for recovering the extractant. In one embodiment, a third distillation column is provided in order to separate off hydrocarbons having more than five carbon atoms. In addition, there is additional equipment required to both prepare and regenerate the solvent for the system. Significant items of equipment are thus required to facilitate the separation. Further, these towers consume significant energy. In all cases, the system operates at low pressures in order to avoid high temperatures in the stripping columns that would degrade the olefin product.

Complete removal of butanes by fractionation or extractive distillation can be costly. In most cases, the cost of this separation is not justified. Fractionation involves the separation of components that have very close boiling points, requiring extensive and expensive fractionation towers. If extractive distillation is used, solvents are employed to reduce tower sizes and utility requirements. Furthermore, the most commonly used extraction solvents contain nitrogen compounds that are significant poisons for isomerization and metathesis catalysts. Typical solvents include NMP and furfural.

It would be useful to develop an efficient and lower cost technique for removing butanes from a metathesis process. Such a process would also be compatible with the metathesis catalyst system and its sensitivity to poisons. It would be also useful to develop an efficient and lower cost technique for removing butanes from a butene isomerization process under conditions that the process would be compatible with the isomerization catalysts.

### Summary of the Invention

An object of the present invention is to provide an efficient method for the removal of butanes from a metathesis process used to produce olefins. This process can be conventional metathesis, autometathesis, or a metathesis process which includes a separate isomerization process, as can be used to produce ethylene and linear alpha olefins.

It is a further object to provide an efficient and low cost method for the removal of butanes from either a double bond or a skeletal isomerization process.

It is still a further object of the invention to provide a method for the removal of butanes from a stream of C4 compounds comprising olefins and paraffins without introducing species that will poison subsequent reaction catalysts.

One embodiment is a process for producing a selected butene, comprising obtaining a C4 feed stream comprising C4 paraffins and C4 olefins, splitting the C4 feed stream to form a first stream comprising a first butene and a second stream comprising a second butene, isomerizing at least a part of the second stream to convert a portion of the second butene to the first butene, and recycling at least some of the isomerized part of the second stream to the splitting step. A portion of at least one of the C4 feed stream and the second stream is passed through a facilitated transport membrane to remove butanes, forming at least one purge stream comprising butanes.

In some cases, the first butene is 1-butene and the second butene is 2-butene. In other cases, the first butene is 2-butene and the second butene is 1-butene. In still other cases, the first butene is normal butene (1 butene and/or 2 butene) and the second butene is isobutylene.

In one form of the invention, the second butene is converted to the first butene by double bond isomerization. In other forms, the second butene is converted to the first butene by skeletal isomerization. In one embodiment, splitting takes place in a catalytic distillation column containing a selective hydrogenation catalyst in which a portion of the 1-butene is converted to 2-butene.

Often, the C4 feed stream contains poisons, and the process further comprises removing poisons from the C4 feed stream before splitting. Sometimes, the C4 feed stream comprises isobutylene, and the process further comprises removing isobutylene from the C4 feed stream before splitting. In certain cases, the C4 feed stream contains at least one of acetylenics and dienes, and the process further comprises removing acetylenics and dienes from the C4 feed stream before splitting.

In all of the embodiments for the production of 1-butene from a second butene (isomerization), either the feed stream or the second stream (before or after isomerization) or both are passed through a membrane system for the selective removal of butanes from the system without the introduction of catalyst poisons.

In some embodiments, the facilitated transport membrane is promoted with at least one of Cu and Ag. Often, the production rate of the first butene product stream is at least 20 % greater, and sometimes 40% greater than the production rate using a process in which splitting and isomerization equipment of approximately equivalent size is used without a facilitated transport membrane.

In many cases, utility consumption is at least 10% lower, or preferably at least 20% lower, than the utility consumption for a process in which approximately equivalent quantities of C4 olefin product are obtained using a C4 feed stream of the same composition and no facilitated transport membrane is included.

Another embodiment is a process for the conversion of C4 olefins, comprising obtaining a C4 feed stream comprising C4 paraffins and C4 olefins, including 1-butene and 2-butene, reacting the C4 feed stream in a metathesis reactor to form a second stream, fractionating the second stream to form one or more product streams containing ethylene, propylene, pentenes and/or hexenes and a recycle stream primarily containing C4 olefins and C4 paraffins, and returning the recycle stream to the metathesis reactor. At least one of the recycle stream and the C4 feed stream is passed through a facilitated transport membrane to remove butanes, forming at least one purge stream comprising butanes.

Sometimes, ethylene is added to the metathesis reactor and the ethylene reacts with 2-butene to form propylene. In some embodiments,the feed to the metathesis reactor is primarily 1-butene and the second stream from the reactor contains ethylene and hexene.

In certain cases, the C4 feed stream contains poisons, and the process further comprises removing poisons from the C4 feed stream before splitting. Sometimes, the C4 feed stream comprises isobutylene, and the process further comprises removing isobutylene from the C4 feed stream before splitting. In some cases, the C4 feed stream contains at least one of acetylenics and dienes, and the process further comprises removing acetylenics and dienes from the C4 feed stream before splitting. The facilitated transport membrane frequently is promoted with at least one of Cu and Ag.

In some embodiments, the production rate of the one or more product streams is at least 10 % greater, or at least 20% greater than the production rate using a process in which approximately equivalent reaction and fractionation equipment is used without a facilitated transport membrane.

Sometimes, the process further comprises isomerizing the C4 feed stream in an isomerization reactor upstream from the metathesis reactor in order to increase the quantity of 1-butene in the C4 feed stream, and fractionating the isomerized C4 feed stream to form a 1-butene rich C4 feed stream and a 2-butene rich bottoms stream. The 2-butene rich bottoms stream can be recycled to the isomerization reactor. The 2-butene rich bottoms stream often is passed through a facilitated transport membrane to remove butanes.

In certain embodiments, the one or more product streams include a hexene product stream, and the process further comprises sending the hexene product stream to a hexene isomerization section to maximize 1-hexene production. Sometimes, the hexene stream is fractionated to form a 1-hexene stream and a mixed 2- and 3-hexene stream, and the mixed 2- and 3-hexene stream is recycled to the hexene isomerization section.

An apparatus for producing a selected butene comprises a butene splitter configured to split a C4 feed stream to form a first stream comprising a first butene and a second stream comprising a second butene, an isomerization reactor configured to isomerize at least a part of the second stream in order to convert at least a portion of the second butene to the first butene, and a facilitated transport membrane configured to remove butanes from at least a portion of one or both of the C4 feed stream and the second stream. The apparatus often has a production capacity at least 20% greater than the production capacity of an apparatus which includes substantially the same splitter and reactor but no facilitated transport membrane.

An apparatus for the conversion of C4 olefins comprises a metathesis reactor, a plurality of fractionation towers positioned downstream from the metathesis reactor for producing a recycle stream and at least one of an ethylene stream, a propylene stream, and a hexene stream, and at least one facilitated transport membrane for removing butanes, the membrane being positioned in at least one of a first location upstream from the reactor and a second location along the recycle stream. In many cases, the apparatus has a production capacity at least 20% greater than the production capacity of an apparatus which includes substantially the same metathesis reactor and plurality of fractionation towers but no facilitated transport membrane.

### Brief Description of the Drawings

Figure 1 is a process flow diagram of one embodiment of a metathesis process according to the present invention.
Figure 2 is a process flow diagram of a second embodiment of the invention.
Figure 3 is a process flow diagram showing a third embodiment of the invention.
Figure 4 is a process flow diagram showing a fourth embodiment of the invention.
Figure 5 is a process flow diagram showing a fifth embodiment of the invention.
Figure 6 is a process flow diagram of a comparative process of 1-butene double bond isomerization in which no membranes are used.
Figure 7 is a process flow diagram for a 1-butene double bond isomerization process in which the feed stream passes through a membrane.
Figure 8 is a process flow diagram for a 1-butene double bond isomerization process in which the recycle stream passes through a membrane.
Figure 9 is a process flow diagram for a metathesis process in which no membrane is used.
Figure 10 is a process flow diagram for a metathesis process in which butanes are removed by extractive distillation instead of using a membrane.
Figure 11 is a process flow diagram for a metathesis process in which the recycle stream passes through a membrane to remove butanes.

### Detailed Description of the Invention

According to the invention, butanes are removed utilizing a membrane system in a metathesis or butene isomerization process. A membrane such as a facilitated transport membrane is employed at one or both of (1) a first location which is in the feed stream upstream from the metathesis reactor and/or fractionation tower, and (2) a second location downstream from the reactor and/or fractionation tower. By removing the butanes at these locations using membranes, a number of advantages can be realized, including a reduction in capital costs, lower energy consumption, a reduction of the number of necessary pieces of operating equipment, an increase in the production rate of products and a reduction in poisons to the reactor system. These advantages can be realized for conventional metathesis in which propylene is produced, autometathesis in which propylene and hexene are produced, a combined isomerization/autometathesis process in which ethylene and hexene are produced, an isomerization process in which 1-butene is produced, an isomerization process in which 2-butene is produced, and a skeletal isomerization process where normal butenes are produced. Production of the desired butenes is increased in all of these cases since a higher recycle rate from the fractionation train can be used and thus a greater percentage of the butenes can be reacted.

As used herein, a "selected" butene is a butene which is a desired product from the C4 stream. Typically, the selected butene is 1-butene, 2-butene, or a mixture of 1-butene and 2-butene.

Without the removal of n-butane and/or isobutane from the recycle stream of the fractionation train, butanes can accumulate in the recycle loops to greater than 70 wt % of the total stream flow. Therefore, any equipment in the recycle loop will be more than double the required size based on non-inert components. Using efficient techniques for butane removal can therefore result in significant cost savings. Removing butane from the feed stream and/or downstream from the metathesis reactor in a metathesis process, and removing butane from the feed stream and/or downstream from the splitter in an isomerization process, substantially reduces equipments costs and energy costs. Butane removal at these locations is done with a facilitated transport membrane.

The metathesis catalyst and the isomerization catalysts are highly susceptible to catalyst poisons. These poisons include oxygenates, sulfur compounds, metals, basic nitrogen compounds, water and highly unsaturated hydrocarbons such as butadiene or vinyl acetylenes that may be present in the C4 streams. Upstream of the metathesis reactor, it is common to employ guard beds. The guard beds contain adsorbent materials that will remove these poisons to very low levels. The guard beds preferably are located after the removal of the highly unsaturated hydrocarbons and remove poisons in the manner described above.

In a process for upgrading C4 streams by metathesis, following the guard beds, if needed, and the removal of isobutylene, if required, the feed stream is sent to metathesis, or isomerization followed by metathesis. In a metathesis reactor, the butenes, and pentenes - if present, are reacted with other olefins, such as ethylene. Typical conversions of the C4 olefins are approximately 60-90 %. As used herein, conversion or utilization of C4 olefins refers to the percentage of 1-butene and 2-butene in the fresh feed stream that is reacted in the isomerization and/or metathesis reactors. The butanes, including both isobutane and n-butane, pass through the reactor system as inerts. The principal product is propylene.

In the process for the production of ethylene and linear alpha olefins, the effluent from the guard beds and isobutylene removal steps can be passed to a high temperature hydrogen free double bond isomerization reactor where the 2-butene is converted to 1-butene. The 1-butene, along with some n-butane and/or isobutane, then passes to metathesis.

In the double bond isomerization process to produce 1-butene, the isobutylene and isobutane often are removed first. The resulting effluent comprising normal butenes and normal butane is then sent to a fractionation tower where some of the 1-butene goes overhead and the 2-butene is a bottoms product. Some 1-butene is also is in the bottoms along with the normal butanes. This bottoms stream is passed through a double bond isomerization reactor to convert 2-butene to 1-butene. The effluent is an equilibrium mixture of 1- and 2-butenes as well as butane. This passes to the fractionation section for recovery of the produced 1-butene.

In a skeletal isomerization process, the C4 feed is sent to a fractionation tower. The overhead stream from a fractionation tower is sent to a skeletal isomerization reaction system. The effluent from that system contains an equilibrium mix of normal and isobutenes. This is recycled to the fractionation tower where the normal butenes are removed as a bottoms product and the isobutylenes (and isobutene) go overhead.

Because butanes are inert with respect to the metathesis or isomerization reactions, they will accumulate within the system. Butane accumulation results in oversized process equipment and larger heating and cooling duties as this component is inert. It also results in the loss of olefins and therefore also a loss of product since butanes must be purged from the system, thus limiting the butane utilization of the process.

Separation of butanes from butenes is difficult. Looking at the boiling points for these C4 components, one can see that the lightest component is isobutylene, followed by isobutane, 1-butene, cis and trans 2-butene, and then n-butane. If both isobutane and n-butane are in the stream, more than one fractionation column is needed to remove them, because valuable olefins are intermediate products. Furthermore, the difference in volatility of these components is very small, thereby necessitating the use of large, energy-intensive columns for separation. If extractive distillation is employed for olefin-paraffin separation, a high percentage of the olefins are recovered from the stream but are contaminated with nitrogen compounds requiring additional guard beds to protect the metathesis catalysts.

Separation of olefins and paraffins using membranes has been discussed in the literature but has not been commercialized to any significant extent because performance of the membranes has not been good enough to replace fractionation as a means of purification and because membranes are not suited for use in situations where components are concentrated but not purified. In order to achieve most commercial purities, membranes are required to be used in combination with fractionation. Furthermore, membranes are sensitive to both temperature and poisons. In many integrated fractionation systems, the temperatures are such that the membranes do not have the mechanical stability for performance.

A newer class of membranes, known as facilitated transport membranes (FTM), are thin membranes which include a carrier species in the membrane that preferentially chemically interacts with a desired component to promote the transfer of the component across the membrane. Typically, facilitated transport membranes use metallic salts impregnated into the membrane or present in liquid layers between two membrane layers to enhance olefin-paraffin separation. The olefin is selectively and weakly absorbed by the membrane and the small (but positive) attractive forces allow for the migration of olefins at a faster rate than the paraffins, thus enhancing the separation factors. For example, olefins but not paraffins have been found to have an attraction to silver cations. Thus, one of the most frequently studied facilitated transport membrane systems uses silver complexes.

Gas permeation across a FTM takes place by two mechanisms: normal solution/diffusion of uncomplexed gas molecules, and diffusion of the carrier-gas complex. The second mechanism occurs only for a gas that reacts chemically with the carrier agent. The total flux across the membrane is the sum of the carrier-gas complex plus the uncomplexed gas flux.

Most FTMs take the form of immobilized liquid membranes (ILM). These are made by impregnating a microporous membrane with a solution of the facilitating carrier in a solvent such as water. The carrier solution is kept within the pores of the support membrane by capillary forces. Alternatively, the carrier liquid can be sandwiched between supporting membrane layers. Examples are described in U.S. Patent Nos. 3,758,603 and 3,758,605. It is very hard to maintain the carrier solution within the membrane and to insure membrane stability, i.e., the carrier does not migrate out of the membrane.

To address these problems of ILMs, solid ion-exchange membranes that exhibit FTM transport of olefins in paraffin-olefins mixtures have been prepared as described in U.S. Patent No. 4,318,714. Another approach is to prepare FTMs from a glassy water soluble polymer such as polyvinyl alcohol to which a complexing water soluble ion of salt such as silver nitrate is added, as is described in U.S. Patent Nos. 5,015,268 and 5,062,866.

U.S. Patent No. 5,670,051 is directed to a facilitated transport membrane in which a metal salt in a polymer matrix interacts with olefins but not paraffins. This document indicates that silver and copper are useful metal ions in the membrane. A preferred approach as taught in U.S. Patent No. 5,670,051 is to prepare a FTM comprised of a solid solution of an ionic metal salt in a polymer, with the membrane being characterized by a selectivity for an unsaturated hydrocarbon such as ethylene or butene over a saturated hydrocarbon such as ethane or butane, respectively, of at least about 20/1 and in a substantially dry environment. The preferred ionic metal is silver, copper, or mixtures thereof. Preferred polymer supports include polyepichlorohydrin, polyether-polyamide block copolymers, epichlorohydrin/ethylene oxide copolymers, polyethylene oxide, and propylene oxide/allyglycidylether copolymers. The pressure normalized flux or permeance of the unsaturated hydrocarbon is said to be at least 10 x 10⁻⁶ cm³ (STP)/cm²/sec/cm Hg. A special unit has been defined as a GPU (Gas Permeance Unit) and is equal to 1 x 10⁻⁶ cm³ (STP)/cm²/sec/cm Hg. Thus the preferred FTM should have a minimum GPU of 10; preferably greater than 20 and most preferably greater than 50. Other FTMs such as those as described in U.S. Patent Nos. 6,468,331; 6,645,276; and 6,706,771 also can be used.

U.S. Patent No. 6,706,771 discloses a silver salt-containing facilitated transport membrane which is said to have good performance over an extended period of time. According to this document, a material is included in the membrane which is able to bind to silver ions in a chelating mode, resulting in improved membrane stability and performance. The membrane comprises a polymer, a silver salt, and a phthalate of the formula C6H4(COOR)2 where R is an alkyl group with 2 - 8 carbon atoms or a phenyl group. The polymer typically contains electron-donating heteroatoms and preferably is a polyvinylpyrrolidone, poly(2-ethyl-2-oxazoline), polyvinylmethylketone, polyvinylformal, polyvinylacetate, cellulose acetate, cellulose acetate butyrate, polyacrylate, polymethylmethacrylate and/or polyacrylic acid. The silver salt preferably is AgBF₄, AgPF₆, AgSO₃CF₃, AgClO₄ and/or AgSbF₆. The phthalate is used in an amount of 0.05 - 10 wt % of the weight of the polymer. Other patent publications including some of the same inventors are U.S. Patent Publication Nos. 2004/0154980, 2001/0015334 and 2002/0162456.

U.S. Patent No. 6,187,196 discloses a facilitated transport membrane which includes first chemical groups that are ionic and are associated with ion exchange sites in the membrane, and a second chemical group which modifies that electronic environment of the ion exchange sites to enhance facilitated transport through the membrane. The first chemical groups preferably are anionic groups for cation exchange, such as a perfluorosulfonic acid polymer, with the ion exchange sites being occupied by a suitable cationic carrier for the facilitated transport. The second chemical group can be, for example, an oxidized poly(pyrrole).

U.S. Patent Publication No. 2004/0147796 discloses a membrane which is said to have high olefin/paraffin selectivity and good durability in long-term contact with hydrocarbon streams. The membrane disclosed therein is said to be particularly useful for separating propylene from mixture or propylene and propane.

It is characteristic of these metal salt promoted membranes that the more unsaturated species (olefins) are preferentially attracted to the metal and hence have a preference for passing through the membrane. More highly unsaturated species such as acetylenics and dienes are even more strongly attracted and in fact bind to the metal salts rendering them inactive to further attraction of olefins. Similarly, sulfur, nitrogen and oxygenate species are strongly polar and will also irreversibly bind to the metallic salts.

As indicated above, the processes disclosed herein often involve the removal of poisons upstream from the facilitated transport membrane. Any trace poisons, especially highly unsaturated hydrocarbons such as dienes and acetylenics, may significantly reduce the performance of the membranes. Compounds such as sulfur and nitrogen are permanent poisons for such membranes. Thus, it is important to remove these materials upstream from the membranes.

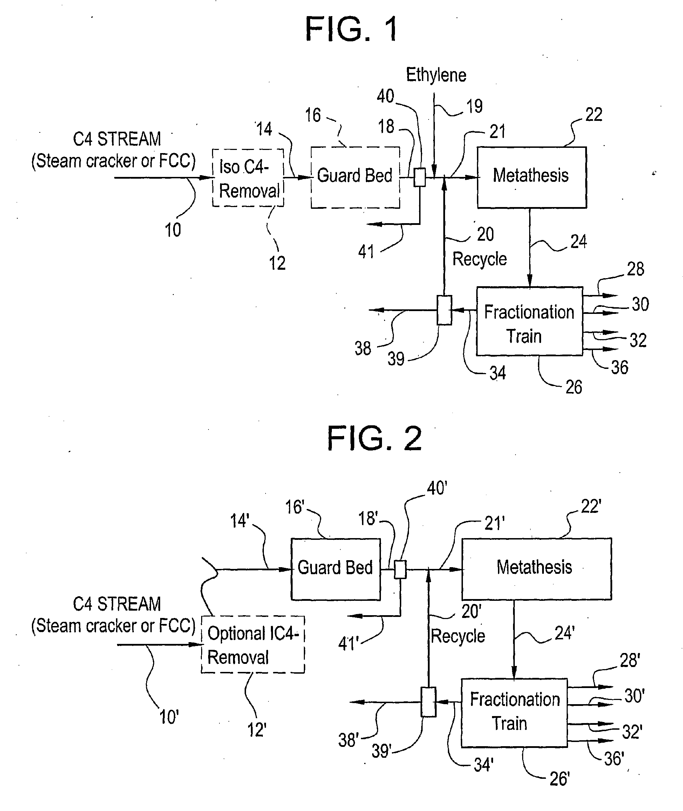

Referring to the drawings and first to Fig. 1, a process flow diagram showing the process of a first embodiment of the present invention is shown. This process is conventional metathesis involving the reaction of ethylene primarily with C4 olefins. A C4 feed stream, designated as 10, is obtained from a steam cracker or FCC unit. The feed stream 10 includes a mix of essentially normal and iso butenes and butanes. In addition small quantities of C3 and C5 components may be present. The highly unsaturated species have been removed from the feed via methods described above. If necessary, the feed stream 10 is fed to an isobutylene removal unit 12, in which the amount of isobutylene is reduced down to no more than 10 wt % of the feed mixture, preferably no more than 5 wt % of the feed mixture and most preferably no more than 2 wt % of the feed mixture. The feed stream with isobutylene removed therefrom is designated as 14. Catalyst poisons are removed from stream 14 in guard beds 16, and the resulting stream 18 optionally passes through a facilitated transport membrane 40 (which will be described below in further detail) in order to remove butanes in stream 41. Stream 18 (or stream 18 minus stream 41 if the membrane 40 is used) is combined with an ethylene stream 19. The flow rate of stream 19 is selected to provide in an appropriate ratio of ethylene relative to the quantity of normal butene in stream 18 (or to the nomal butene content of stream 18 minus stream 41) for the metathesis reactor. A recycle stream 20 from downstream purification is also combined with these streams and the combined streams are fed as stream 21 to a metathesis reactor 22.

In the metathesis reactor 22, the olefins react with each other to produce a reactor effluent 24 containing ethylene, propylene, unreacted butenes, pentenes and higher olefins. In addition, any paraffins in the feed pass through the reactor as inerts.

The metathesis reactor 22 usually operates at a pressure between 2 and 40 atmospheres and preferably between 5 and 25 atmospheres. The catalyst contained within this reactor may be any suitable metathesis catalyst including but not limited to oxides of Group VIB and Group VII B metals on supports. Catalyst supports can be of any type and could include alumina, silica, mixtures thereof, zirconia, and zeolites. In addition to the metathesis catalyst, the catalyst in reactor 22 can include a double bond isomerization catalyst such as magnesium oxide or calcium oxide. The reaction takes place at a temperature between 50° and 450° C, preferably between 300° and 400° C.

Downstream of the metathesis reactor 22 is a fractionation train 26 consisting primarily of fractionation towers. Ethylene is separated in a first tower in line 28 and can be recycled to the reaction zone where it is combined with stream 19. Propylene is produced as a product in line 30 from a second tower. The unreacted butenes as well as the C4 paraffins and any pentenes and heavier olefins formed in the reaction are the bottom products from the propylene separation. It is not uncommon to employ additional towers to separate the C4 compounds as stream 34 from the heavier compounds such as pentenes in stream 32 and higher olefins in stream 36 and recycle the C4 compounds to increase the overall utilization of the olefins. Alternately, the C4 stream could be obtained by using a side draw from the propylene separation tower. In this case the heavy components in stream 36 are the bottoms product from the propylene separation. The recycle C4 stream 34 (either from a separate tower or as a side stream withdrawn from the propylene separation tower), contains the inert C4 paraffins. These must be removed in order to maximize the recovery of the butenes or the volume of material recycled will become very large requiring substantial equipment and thus costs.

While it is possible to remove the paraffins before the metathesis reactor 22 or in the recycle stream 34 by fractionation or extractive distillation, fractionation is very costly both in terms of equipment costs and energy due to the close boiling points of the components and extractive distillation is also costly due to the large number of towers required as well as solvent preparation systems. Further, extractive distillation re-introduces poisons into the system. A better method for separation is required. Membranes, including facilitated transport membranes, represent such a system. Since facilitated transport membranes are also poisoned by highly unsaturated compounds as well as oxygenate and sulfur compounds, they are ideally suited to use within a metathesis loop where the catalyst is also sensitive to the same contaminants.

The facilitated transport membrane is ideally located in the recycle stream 34 following the metathesis reactor. Such a membrane is shown in Fig. 1 as membrane 39. After the reaction step, the olefins concentration within the C4 stream is reduced. Thus the quantity of material that has to pass through the membrane is reduced thus both improving its performance and reducing costs. In addition to or instead of membrane 39, membrane 40 can be used in the feed stream, as indicated above.

A stream 38 is separated from the C4 stream 34 and is used to control the amount of recycle to avoid overloading the reactor. The stream 38 is a predominately butane stream that is obtained using the facilitated membrane 39 which separates paraffins from olefins and therefore separates butanes from butenes.

The recycle stream 20, which is the butene/butane stream 34 with the butane purge stream 38 removed therefrom, is combined into the metathesis feed stream with ethylene added, the combined stream being shown as stream 21. By removing butane without simultaneously removing butane in membranes 39 and/or 40, a higher conversion of butenes to propylene is achieved.

Fig. 2 depicts a second embodiment in which the metathesis process is autometathesis, which does not involve the addition of ethylene. In this process, butane removal by a membrane such as a facilitated transport membrane can take place at one or both of the same locations as in the embodiment for Fig.1, namely in the feed stream 18' or in the C4 recycle stream 34'. In Fig. 2, the membranes are shown as 39' and 40'. In this case, the C4s react with themselves by autometathesis to form principally ethylene, propylene, pentenes and hexenes. The isobutylene may or may not be removed upstream form the guard beds, depending upon the product desired. Furthermore, selective hydrogenation can take place in stream 10' or 14'.

The feed stream 10' optionally is subjected to isobutylene removal in isobutylene removal unit 12' and optionally is conveyed in stream 14' to a guard bed 16' for removal of poisons. The purified stream 18' optionally passes through membrane 40' to remove butanes in a purge stream 41'. The remaining feed stream 21' enters an autometathesis reactor 22' in which ethylene, propylene, pentenes and hexenes are formed and exit the reactor 22' in stream 24' along with remaining C4s. Downstream from the autometathesis reactor 22' is a fractionation train 26' consisting primarily of fractionation towers. Ethylene is separated in a first tower in line 28'. It can be recovered as a product or recycled to the reaction zone. Propylene is produced as a product in stream 30'. In some cases, a third tower separates the C4 compounds from heavier compounds. The C4 compounds are recycled in stream 34'. The C5 compounds are removed in stream 32'. The heavier compounds are removed in stream 36'. The C4 stream 34' optionally passes through a membrane 39' to remove butanes in a butane purge stream 38', with the remaining recycle forming stream 20'.

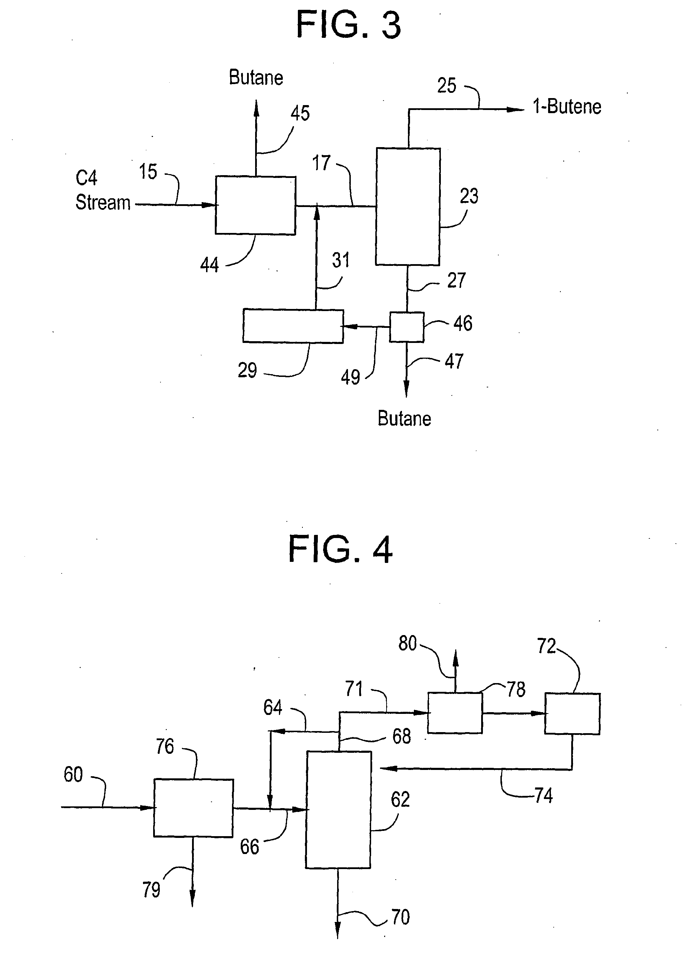

Figure 3 shows a 1-butene double bond isomerization process in which a feed stream 15 containing C4 paraffins and C4 olefins is combined with a recycle stream 31 to form stream 17. In order to produce high purity 1-butene, stream 15 should contain only minor amounts of isobutylene and isobutane. An upstream fractionation system would be employed to remove the majority of these iso compounds simultaneously if there is a high concentration of these materials. Stream 17 is split in a splitter 23 to separate the mixture into an overhead stream 25 comprising 1-butene and a bottoms stream 27 comprising 2-butene and normal butane. The 2-butene is isomerized in an isomerization reactor 29 to form the recycle stream 31, which is combined with the fresh feed to form stream 17. All or part of stream 27 can be recycled through the double bond isomerization reactor 29. One or both of membranes 44 and 46 are included to remove butanes. Membrane 44 is positioned in the feed stream 15 and removes butanes in stream 45. A membrane at this location will remove both any remaining isobutane and normal butane. A membrane at this location which removes the isobutane will increase the purity of the 1-butene since the isobutane would track with the 1-butene and go overhead in the fractionation column. Membrane 46 is positioned between the splitter 23 and the isomerization reactor 29 and removes butanes in stream 47, while remaining stream 49 enters the isomerization reactor 29. A membrane at this location removes primarily normal butane and reduces the hydrocarbon flow through the system by reducing the buildup of butane in the recycle.

Figure 4 shows a butene skeletal isomerization process in which feed stream 60 containing C4 olefins and paraffins is fed to a fractionation tower 62 after being combined with recycle stream 64. The combined stream 66 is separated into an overhead stream 68 comprising isobutane and isobutene and a bottoms stream 70 comprising normal butane and normal butenes (both 1-butene and 2-butene). A portion of the overhead in stream 71 is sent to a skeletal isomerization reaction system 72 where isobutene is converted to normal butene. The equilibrium mixture of normal and isobutene and isobutane is recycled to the fractionation tower 62 in stream 74. One or two membranes (76 and 78) are included to remove butanes. A membrane 76 positioned in the feed stream removes both isobutane and normal butane. The butane purge stream 79 removes the butanes as a product. A membrane 78 positioned in the overhead stream 71 will reduce the isobutane content of the recycle feed through the skeletal isomerization reaction system 72. Butanes are removed in stream 80. Both membranes will reduce the equipment sizes and utility consumptions for a given production.

To produce 1-butene, bottoms stream 70 can be subsequently subjected to double bond isomerization using the process of Figure 3. To produce 2-butene from stream 70, there are two options. The first is to subject stream 70 to hydroisomerization in a fixed bed, a fixed bed with a butene splitter recycle, a fixed bed followed by a catalytic distillation column, or a single tower with both catalytic distillation and hydroisomerization. The other option is to put a catalytic distillation section in fractionation tower 62, which will increase the percentage of 2-butenes in stream 70.

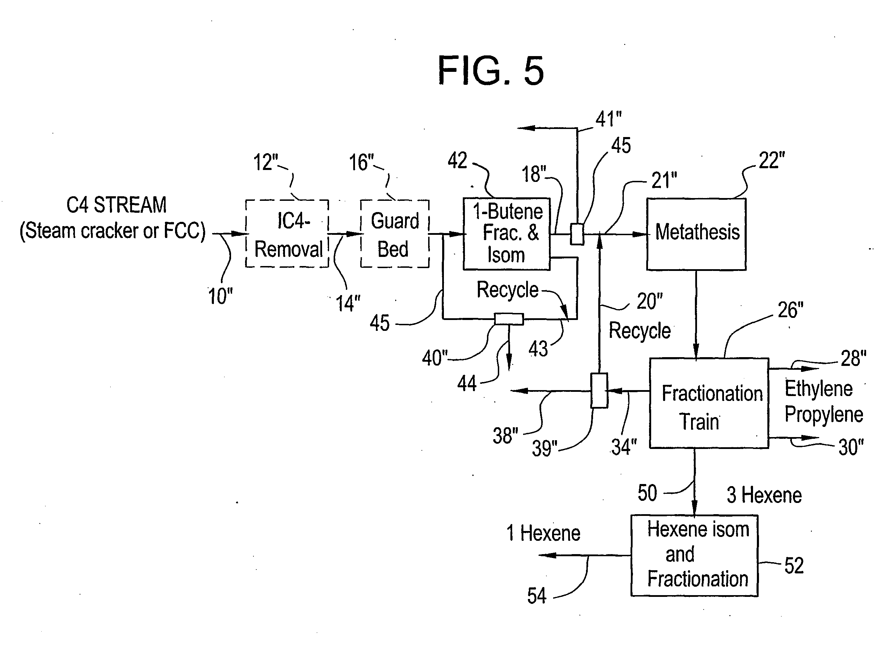

Fig. 5 shows an embodiment in which ethylene and linear alpha olefins are produced by metathesis, with a separate isomerization reactor 42 included upstream from the metathesis reactor 22". One or more of three different membranes, designated as 39", 40" and 45, are included. In this case, isobutylene (if present) is removed from feed stream 10" to a very low level, e.g. about 0.03% in isobutylene removal unit 12". By limiting the amount of iso-olefins in the feed to the metathesis reactor 22", the quantity of branched olefins in the effluent is minimized. From the isobutylene removal unit 12", the feed is conveyed in stream 14" to a guard bed 16" for removal of poisons. The feed is isomerized in a reactor 42, optionally passed through a membrane 45, to remove butanes, and then conveyed to the metathesis reactor 22" in which ethylene, propylene, pentenes and hexenes are formed and exit the reactor 22" with the remaining C4s in stream 24". In the fractionation train 26", ethylene and propylene are removed in streams 28" and 30", respectively. The 3-hexene in stream 50 is isomerized and fractionated at 52 in order to form 1-hexene product stream 54. Butane optionally is removed from C4 stream 34" by membrane 39", which preferably is a facilitated transport membrane, in the recycle loop downstream from the fractionation train 26" as in the first and second embodiments, and/or using membrane 40" in stream 43, which constitutes the recycle loop for the reactor 42. When membrane 45 is used, butane is removed in stream 41". When membrane 39" is used, butane is removed in stream 38". When membrane 40" is used, butane is removed in stream 44. In this case, ethylene, propylene and 1-hexene are the products, with 3-hexene being isomerized, followed by fractionation to obtain 1-hexene. The advantages of butanes (iso or normal or both) removal at the locations shown in Figs. 1-5 are as follows:
○ In general, by removing the butanes from the C4 stream, the volume of material to be processed has been reduced substantially. This saves capital cost. In many cases but not all, for metathesis, the removal of isobutylene is also desired. For conventional metathesis ethylene is added and thus one should remove isobutylene to a low amount (2-5%). For, hexene autometathesis isobutylene usually should be removed to 0.03%. For general autometathesis there is no requirement to remove isobutylene. However, depending upon the method of isobutylene removal, isobutane can remain, since it is an inert. If a fractionation option is used to remove isobutylene, then isobutane is removed in addition to the isobutylene. This further reduces costs. If however MTBE or dimerization is used, isobutane remains and removal via a membrane is advantageous.
○ Normal butane is the least volatile of the C4 paraffins and isobutane is the most volatile. The normal olefins (1 and 2 butene) boil in between these two paraffins. If both paraffins (iso and normal) are present, a membrane can remove both simultaneously. Fractionation is expensive and difficult due to the close volatility. Further, fractionation needs multiple towers since isobutane is the most volatile and normal butane is the least volatile. Extractive distillation will remove both paraffins simultaneously but is also very expensive and will introduce poisons into the system that are detrimental to the metathesis catalysts.
○ Typically the C4 recycle stream after metathesis is a side draw from the lower portion of a tower used to separate propylene from heavier material in the fractionation section. The C5 and heavier material produced in the metathesis reaction is a bottoms product. The C3 is the overhead product in this tower. The C4 stream after the reaction at this point has lower olefins than upstream of the reactor and also has lower volume (since olefins have been reacted away to other carbon number olefins). By starting with a more concentrated stream (of butanes), the separation is easier and less costly.
○ The C4 stream has already passed through a guard bed to remove poisons and the metathesis reactor that has additionally removed any poisons (by fouling, although this is not desired). For any membrane separation option, the recycle feed is usually the most suitable. The removal of poisons will provide the longest membrane life at the best performance. This is especially true for the facilitated transport membrane systems.
○ For those systems with isobutane, and especially refinery feedstocks that have high isobutylene and isobutane and where the isobutylene has been removed via dimerization or MTBE (to make gasoline octane components), the use of an olefin paraffin membrane is especially critical to achieve economic butene utilization. Furthermore, refinery type feedstocks have a higher level of poisons (sulfur and oxygenates) than steam cracker feedstocks. By locating the membrane-based butane removal step downstream of guard beds and the primary reaction, a major reduction in capital costs can be achieved. Further, facilitated membranes are the best option for membrane removal and require the cleanest service.
○ By effectively removing the butanes, the yield of the desired olefins from isomerization (for example 1 butene from 2 butene) or from metathesis (for example propylene from conventional metathesis) can be increased since a higher recycle rate can be used before experiencing severe equipment limitations and thus a greater percentage of the butenes can be reacted.

The following examples illustrate important features of the invention.

### Comparative Example 1

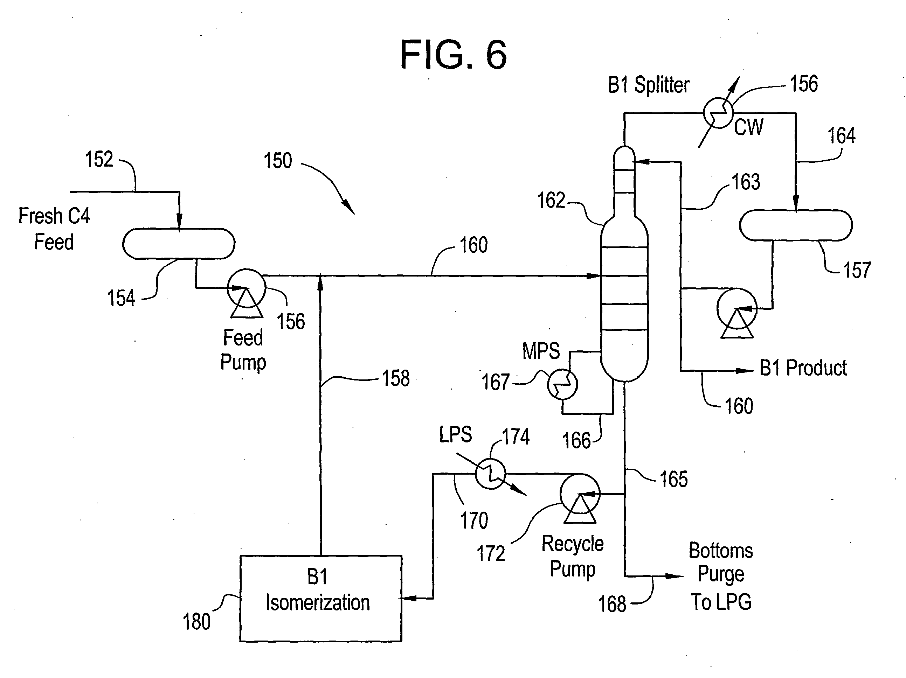

A sophisticated computerized simulation of a 1-butene isomerization process flow scheme using the process and equipment shown in Fig. 6 was conducted. The overall process is designated as 150. Fresh C4 feed in stream 152, from which isobutylene and isobutane have been removed, was fed to a surge tank 154 for a feed pump 156. The pump 156 conveyed the fresh C4 feed, which was combined with a 1-butene isomerization reactor effluent stream 158 in stream 160, into a 1-butene splitter 162. 1-Butene was removed from the top of the splitter 162 in stream 164. The top stream 164 was condensed in a condenser 156, fed to a tank 157, and separated into a 1-butene product stream 160 and a recycle stream 163, both of which were conveyed by a pump 164. The 2-butene was removed from the splitter 162 as bottoms in stream 165. Stream 166 was removed from the bottom of the splitter 162, reboiled in reboiler 167, and injected into the bottom of the splitter 162. Stream 165 was separated into a purge stream 168 and a recycle stream 170. Recycle stream 170 was conveyed by pump 172 through a heat exchanger 174 and to a 1-butene isomerization section 180 in which 2-butene was converted to 1-butene. The reactor effluent stream 158 was combined with fresh feed in stream 152 to form stream 160. The process had a 90% utilization of normal butenes in the fresh feed, i.e. 90% of the 1-butene and 2-butene in the fresh feed was reacted.

The compositions for the feed, product and purge streams for the process of Fig. 6 are shown in Table 1 below. An overall material balance and utilities summary is shown in Table 4.

**Table 1: Comparative Example 1**

| Component | FRESH C4 FEED | BOTTOMS PURGE | B1 PRODUCT |
|---|---|---|---|
| | kg/hr | kg/hr | kg/hr |
| i-Butane | 0.0 | 0.0 | 0.0 |
| 1-3BD | 0.0 | 0.0 | 0.0 |
| n-Butane | 964.1 | 952.4 | 17.4 |
| NButenes | 2913.0 | 275.2 | 2637.4 |
| iButenes | 5.9 | 0.0 | 5.9 |
| Heavies | 117.1 | 111.8 | 0.0 |
| Total | 4000.1 | 1339.4 | 2660.7 |

### Example 1

The process of Comparative Example 1 was repeated with the exception that downstream from the feed stream pump 256, fresh C4 feed stream 252, from which isobutylene and isobutane have been removed, was heated in a heat exchanger 255 and passed through a facilitated transport membrane 253 in order to remove butanes in a retentate stream 257. The retentate stream 257 was removed from the system as a purge stream. The purified C₄ feed stream 259 was combined with a 1-butene isomerization reactor effluent stream 258 to form stream 260, which was fed to a 1-butene splitter 262.

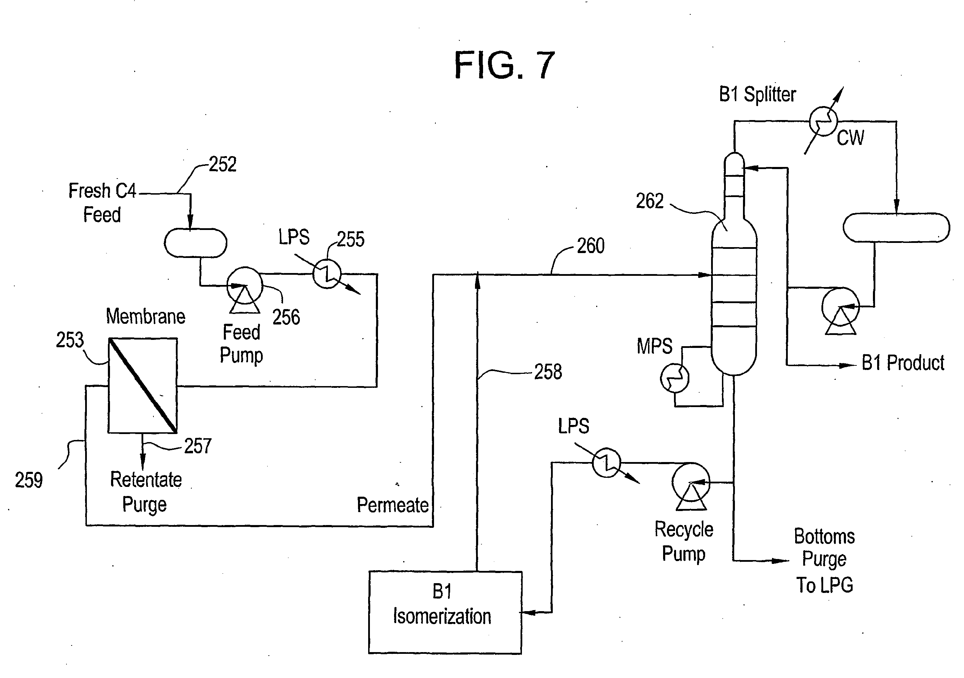

The compositions for the feed, permeate, retentate, splitter bottoms purge, and product streams for the process of Fig. 7 are shown below in Table 2. The overall material balance and utilities summary is shown in Table 4.

**Table 2: Example 1**

| Component | FRESH C4 FEED | PERMEATE | RETENTATE PURGE | BOTTOMS PURGE | B1 PRODUCT |
|---|---|---|---|---|---|
| | kg/hr | kg/hr | kg/hr | kg/hr | kg/hr |
| i-Butane | 0.0 | 0.0 | 0.0 | 0.0 | 0.0 |
| 1-3BD | 0.0 | 0.0 | 0.0 | 0.0 | 0.0 |
| n-Butane | 964.1 | 246.8 | 717.3 | 236.6 | 12.0 |
| NButenes | 2913.0 | 2885.1 | 27.8 | 244.3 | 2640.5 |
| iButenes | 5.9 | 5.9 | 0.1 | 0.0 | 5.9 |
| Heavies | 117.1 | 30.0 | 87.1 | 28.3 | 0.0 |
| Total | 4000.1 | 3167.7 | 832.3 | 509.2 | 2658.3 |

### Example 2

The process of Comparative Example 1 was repeated with the exception that between the heat exchanger 374 and the 1-butene isomeriztion section 380, the 1-butene splitter recycle stream 370 was passed through a facilitated transport membrane 353 in order to remove in a retentate stream 355. The retentate stream 355 was removed from the system as a purge stream. The purified recycle stream 376 was compressed in a compressor 357 and conveyed to the 1-butene isomerization section 380. The overall process is designated as 350.

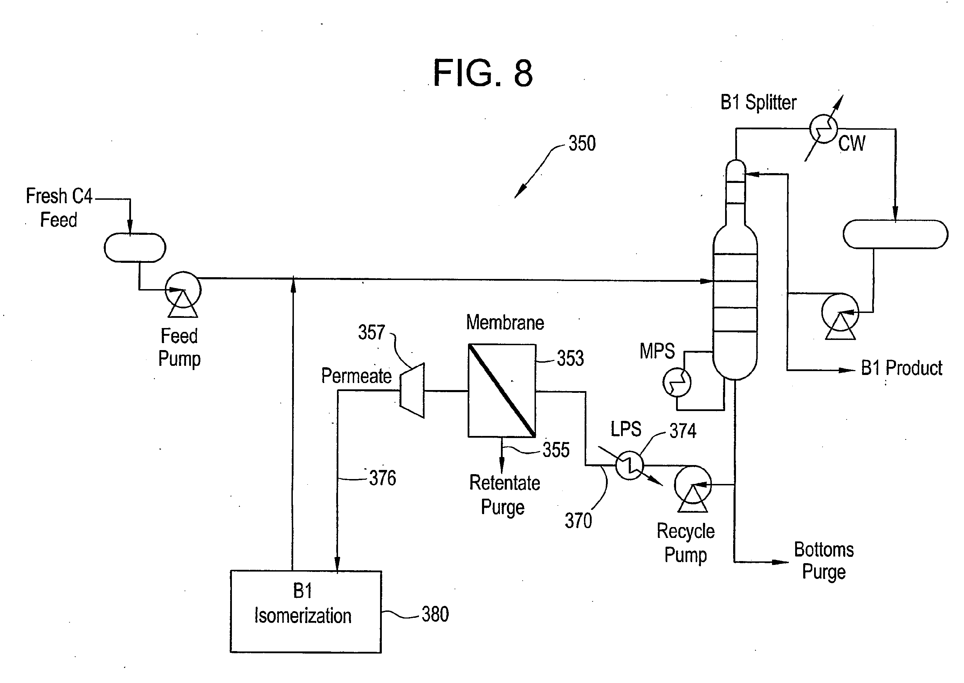

The compositions for the feed, permeate, retentate, splitter bottoms purge and product streams for the process of Fig. 8 are shown on Table 3. The overall material balance and utilities summary is shown on Table 4.

**Table 3: Example 2**

| Component | FRESH C4 FEED | PERMEATE | RETENTATE PURGE | BOTTOMS PURGE | B1 PRODUCT |
|---|---|---|---|---|---|
| | kg/hr | kg/hr | kg/hr | kg/hr | kg/hr |
| i-Butane | 0.0 | 0.0 | 0.0 | 0.0 | 0.0 |
| 1-3BD | 0.0 | 0.0 | 0.0 | 0.0 | 0.0 |
| n-Butane | 964.1 | 59.6 | 955.0 | 6.6 | 3.0 |
| NButenes | 2913.0 | 8147.7 | 215.2 | 56.9 | 2640.4 |
| iButenes | 5.9 | 0.1 | 0.0 | 0.0 | 5.9 |
| Heavies | 117.1 | 7.2 | 115.4 | 1.7 | 0.0 |
| Total | 4000.1 | 8214.5 | 1285.6 | 65.2 | 2649.3 |

**Table 4: Overall Material Balance and Utilities Summary**

| | Comparative Example 1 | Example 1 | Example 2 |
|---|---|---|---|
| | NO MEMBRANE | MEMBRANE CASE | MEMBRANE CASE |
| UTILIZATION, % | 90 | 90 | 90 |
| | | | |
| | | | |
| | | | |
| | | | |

| Feeds, MTA | | | |
|---|---|---|---|
| Fresh C4 Feed | 32,000 | 32,000 | 32,000 |
| Total Feed, MTA | 32,000 | 32,000 | 32,000 |
| | | | |

| Products, MTA | | | |
|---|---|---|---|
| | | | |
| Butene 1 Product | 21,288 | 21,265 | 21,192 |
| Bottoms Purge to LPG | 10,712 | 4,074 | 520 |
| Retentate Purge to LPG | 0 | 6,661 | 10,288 |
| Total Products, MTA | 32,000 | 32,000 | 32,000 |
| | | | |

| Utilities | | | |
|---|---|---|---|
| CW, MMKCAL/Hr | 30.950 | 12.330 | 6.361 |
| MP Steam, MMKcal/Hr | 26.490 | 10.460 | 5.148 |
| LP Steam, MMKcal/Hr | 2.740 | 1.370 | 0.843 |
| Power, MMKcal/Hr | 0.005 | 0.002 | 0.272 |
| Fuel, MMKcal/Hr | 1.790 | 0.629 | 0.069 |
| | | | |
| Total, MMKcal/Hr | 61.98 | 24.79 | 12.69 |
| | | | |

| Major Equipment Sizing | | | |
|---|---|---|---|
| Tower | | | |
| Tower Diameter, m (Top/Bottom) | 4.88/5.18 | 2.89/3.20 | 2.13/2.44 |
| | | | |
| B1 ISOM Reactor | | | |
| Length X Diameter, m/m | 3.66x1.68 | 3.66x1.07 | 3.66x0.91 |
| | | | |
| Recycle Pump | | | |
| Liquid Flow Rate, GPM | 445 | 158 | 82 |
| | | | |
| Reflux Pump | | | |
| Liquid Flow Rate, GPM | 3182 | 1267 | 654 |
| | | | |

| Membrane Area | | | |
|---|---|---|---|
| Membrane 1, m2 | na | 9422 | 3,935 |
| Total, m2 | 0 | 9,422 | 3,935 |

### Example 3

The process of Example 2 was repeated except that the 1-butene isomerization capacity was increased by 40%. The results are shown on Table 5. This Example shows the advantages of membranes in both improving capacity and reducing utility consumption. Even with the higher capacity operation, the sizes of the equipment and the recycle and reflux flows (hence pumping requirements) are still lower than the case of comparative example 1 and the utilities are 71 % lower than the case of comparative example 1.

**Table 5: OVERALL MATERIAL BALANCE AND UTILITIES SUMMARY**

| | Comparative Example 1 | Example 2 | Example 3 |
|---|---|---|---|
| | NO MEMBRANE | MEMBRANE CASE | MEMBRANE CASE |
| UTILIZATION, % | 90 | 90 | 90 |
| | | | |
| | | | |
| | | | |
| | | | |

| Feeds, MTA | | | |
|---|---|---|---|
| Fresh C4 Feed | 32,000 | 32,000 | 44,800 |
| Total Feed, MTA | 32,000 | 32,000 | 44,800 |
| | | | |

| Products. MTA | | | |
|---|---|---|---|
| | | | |
| Butene 1 Product | 21,288 | 21,192 | 29,518 |
| Bottoms Purge to LPG | 10,712 | 520 | 2,146 |
| Retentate Purge to LPG | 0 | 10,288 | 13,136 |
| Total Products, MTA | 32,000 | 32,000 | 44,800 |
| | | | |

| Utilities | | | |
|---|---|---|---|
| CW, MMKCAL/Hr | 30.950 | 6.361 | 8.897 |
| MP Steam, MMKcal/Hr | 26.490 | 5.148 | 7.206 |
| LP Steam, MMKcal/Hr | 2.740 | 0.843 | 1.162 |
| Power, MMKcal/Hr | 0.005 | 0.272 | 0.380 |
| Fuel, MMKcal/Hr | 1.790 | 0.069 | 0.372 |
| | | | |
| Total, MMKcal/Hr | 61.98 | 12.69 | 18.02 |
| | | | |

| Major Equipment Sizing | | | |
|---|---|---|---|
| Power | | | |
| Power Diameter, | 4.88/5.18 | 2.13/2.44 | 2.60/2.74 |
| m (Top/Bottom) | | | |
| B1 ISOM Reactor | | | |
| Length X Diameter, m/m | 3.66x1.68 | 3.66x0.91 | 3.66x0.93 |
| | | | |
| Recycle Pump | | | |
| Liquid Flow Rate, GPM | 445 | 82 | 113 |
| | | | |
| Reflux Pump | | | |
| Liquid Flow Rate, GPM | 3182 | 654 | 914 |
| | | | |

| Membrane Area | | | |
|---|---|---|---|
| Membrane 1, m2 | na | 3,935 | 6,894 |
| Total, m2 | 0 | 3.935 | 6,894 |

### Comparative Example 4A

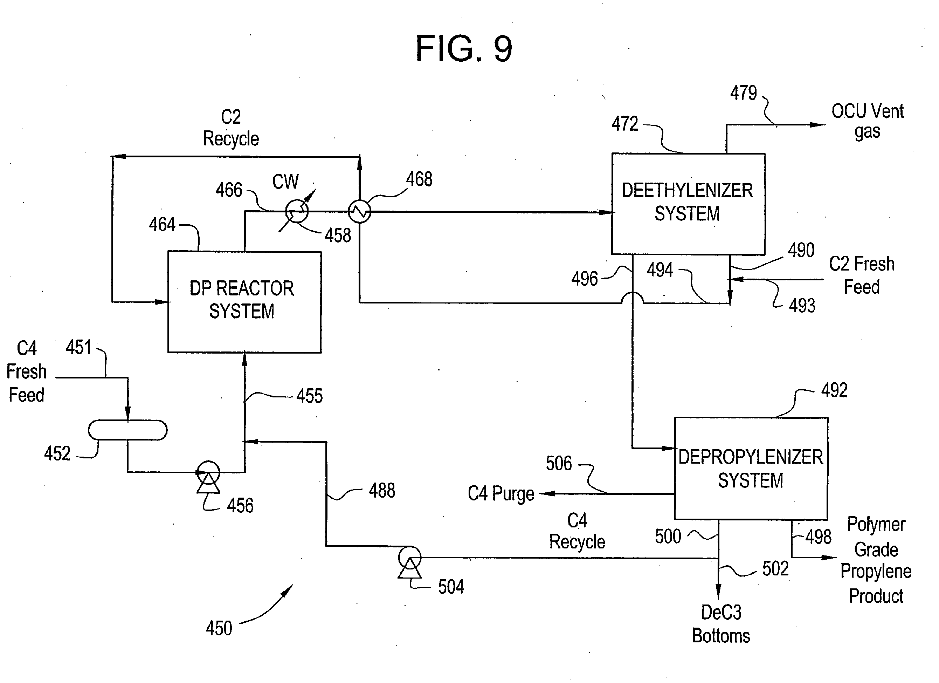

A sophisticated computerized simulation of an optimized metathesis process using the equipment and process flow scheme shown in Fig. 9 was run. The overall process is designated as 450 and is designed to result in 90% utilization of the 1- and 2-butene in the fresh feed. Fresh C4 feed in stream 451 was fed to a surge tank 452 for a pump 456, and was combined with C2 recycle from stream 488 in stream 455. Stream 455 was fed to a metathesis reactor system 464. The metathesis product was passed as stream 466 through a heat exchanger 458 where it was cooled, and through another heat exchanger 468, where it was further cooled. The cooled metathesis product stream 466 was then fed to a deethylenizer system 472.

C2s were removed from the top of the deethylenizer system 472 as vent gas stream 479. The C3s and heavier materials from the deethylenizer system 472 were removed in bottoms stream 496. Bottoms stream 496 was fed to a depropylenizer system 492. Recycle stream 490 from the deethylenizer system 472 was combined with fresh C₂ in stream 493 to form stream 494. Stream 494 was heated in heat exchanger 468 and recycled to the metathesis reactor system 464.

In the depropylenizer system 492, polymer grade propylene product was removed in stream 498. The bottoms were removed as stream 500, which was divided into a depropylenizer bottoms stream 502 and the C4 recycle stream 488. Pump 504 conveyed the recycle stream 488 to be combined with feed stream 454 to form stream 455. A second C4 purge was removed from the middle of depropylenizer 492 as stream 506.

A material balance for the process of Fig. 9 is shown below in Table 6 and in material balance summary table 9. The utility consumption and equipment piece count are shown in Table 10 below.

**Table 6: Comparative Example 4A**

| Component | C4 Fresh Feed | C2 Fresh Feed | C2 Recycle | C4 Recycle | DeC3 Bottom | PG Propylene Product | C4 Purge | OCU Vent Gas |
|---|---|---|---|---|---|---|---|---|
| | Kg/H | Kg/H | Kg/H | Kg/H | Kg/H | Kg/H | Kg/H | Kg/H |
| Methane | 0.0 | 3.4 | 626.9 | 0.0 | 0.0 | 0.0 | 0.0 | 3.3 |
| Ethylene | 0.0 | 11439.3 | 34114.5 | 0.0 | 0.0 | 0.5 | 0.0 | 50.1 |
| Ethane | 0.0 | 5.7 | 31.7 | 0.0 | 0.0 | 5.6 | 0.0 | 0.0 |
| Propene | 0.0 | 0.0 | 534.0 | 27.1 | 27.1 | 34107.4 | 133.6 | 0.2 |
| Propane | 0.0 | 0.0 | 0.0 | 0.0 | 0.0 | 0.0 | 0.0 | 0.0 |
| i-Butane | 6970.9 | 0.0 | 0.0 | 20175.8 | 20171.9 | 2.1 | 6972.6 | 0.0 |
| 1-3BD | 0.0 | 0.0 | 0.0 | 0.0 | 0.0 | 0.0 | 0.0 | 0.0 |
| n-Butane | 1529.3 | 0.0 | 0.0 | 7109.2 | 7108.5 | 0.0 | 1530.0 | 0.0 |
| N- Butenes | 24972.4 | 0.0 | 0.0 | 8718.3 | 8719.3 | 0.5 | 1993.5 | 0.0 |
| i-Butenes | 16.8 | 0.0 | 0.0 | 48.7 | 48.7 | 0.0 | 15.4 | 0.0 |
| Heavies | 0.0 | 0.0 | 0.0 | 1241.3 | 1241.5 | 0.0 | 123.1 | 0.0 |
| Total | 33489.4 | 11448.4 | 35307.1 | 37320.4 | 37317.0 | 34116.1 | 10768.2 | 53.6 |

| | | | | | | | | |
|---|---|---|---|---|---|---|---|---|
| PG = Polymer Grade OCU = Olefins conversion unit | | | | | | | | |

### Comparative Example 4B

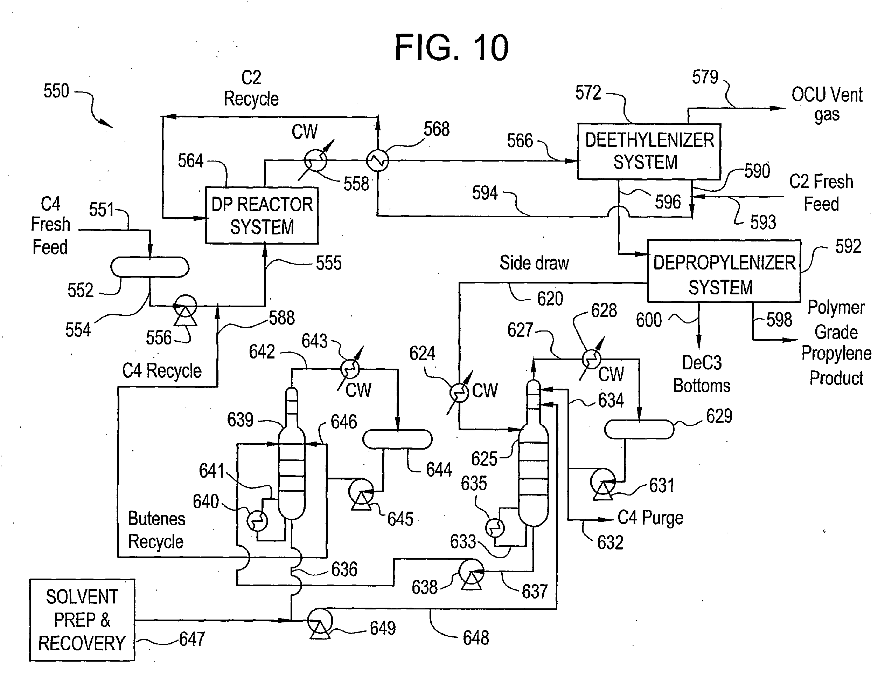

A sophisticated computerized simulation of a metathesis process using the equipment and process flow scheme shown in Fig. 10 was run.

The overall process is designated as 550. Fresh C4 feed in stream 551 was fed from a feed drum 552 in stream 554 by pump 556, and was combined with C2 recycle from stream 588 into stream 555. Stream 555 was fed to a metathesis reactor system 564. The metathesis product was passed as stream 566 through heat exchanger 558 where it was cooled, and through another heat exchanger 568 where it was further cooled. The cooled metathesis product stream 566 was fed to deethylenizer system 572.

C2s were removed from the top of the deethylenizer system 572 as a vent gas stream 579. The C3s and heavier materials from the deethylenizer system 572 were removed in bottoms stream 596. Bottoms stream 596 was fed to a depropylenizer system 592. Recycle stream 590 from the deethylenizer system 572 was combined with fresh C2 feed in stream 593 to form stream 594. Stream 594 was heated in heat exchanger 568 and recycled to the metathesis reactor system 564.

In the depropylenizer system 592, polymer grade propylene product was removed in stream 598. The bottoms were removed as stream 600. A side draw was removed in stream 620.

The side draw 620 was cooled in heat exchanger 624 and then passed through an extractive distillation column 625. The top stream 627 was cooled in a condenser 628, sent to a drum 629, and conveyed by a pump 631. Stream 627 was divided into a C4 purge stream 632 and a recycle stream 634 back to column 625. Part of the bottoms from the extractive distillation column 625 was removed in stream 633, reboiled in reboiler 635 and introduced back into the column 625. Most of the bottoms from the extractive distillation column was removed in stream 637 and conveyed by pump 638 to a stripper column 639, which had a reboiler 640 for reboiler stream 641. The top stream 642 from the stripper column 639 was condensed in a condenser 643, sent to a drum 644, and conveyed by a pump 645 partially back to the stripper column in stream 646 and partly in stream 588 back to be mixed with fresh feed from stream 554. A solvent preparation and recovery system 647 provided a solvent stream 648 to the extractive distillation column 625 via pump 649. The bottoms stream 636 containing recovered solvent from the stripper 639 was combined with stream 647 to form stream 648.

A material balance for the process of Fig. 10 is shown below on Table 7 and in a material balance summary on Table 9. The utility consumption and equipment piece count are shown in Table 10 below.

**Table 7: Comparative Example 4B**

| Component | C4 Fresh Feed | C2 Fresh Feed | C2 Recycle | C4 Recycle | DeC3 Bottom | PG Propylene Product | C4 Purge | OCU Vent Gas |
|---|---|---|---|---|---|---|---|---|
| | Kg/Hr | Kg/Hr | Kg/Hr | Kg/Hr | Kg/Hr | Kg/Hr | Kg/Hr | Kg/Hr |
| Methane | 0.0 | 6.2 | 1116.2 | 0.0 | 0.0 | 0.0 | 0.0 | 4.6 |
| Ethylene | 0.0 | 14386.6 | 42778.9 | 0.0 | 0.0 | 0.4 | 0.0 | 47.8 |
| Ethane | 0.0 | 7.2 | 87.8 | 0.0 | 0.0 | 7.1 | 0.0 | 0.1 |
| Propene | 0.0 | 0.0 | 679.1 | 0.4 | 0.0 | 43027.1 | 0.0 | 0.2 |
| Propane | 0.0 | 0.0 | 0.0 | 0.0 | 0.0 | 0.0 | 0.0 | 0.0 |
| i-Butane | 8326.2 | 0.0 | 0.0 | 89.8 | 46.8 | 0.8 | 8279.9 | 0.0 |
| 1-3BD | 0.0 | 0.0 | 0.0 | 0.0 | 0.0 | 0.0 | 0.0 | 0.0 |
| n-Butane | 1826.6 | 0.0 | 0.0 | 19.8 | 12.0 | 0.0 | 1814.6 | 0.0 |
| N- Butenes | 29827.6 | 0.0 | 0.0 | 12420.2 | 87.1 | 0.6 | 1027.0 | 0.0 |
| i-Butenes | 20.1 | 0.0 | 0.0 | 37.6 | 0.3 | 0.0 | 19.5 | 0.0 |
| Heavies | 0.0 | 0.0 | 0.0 | 1699.6 | 24.1 | 0.0 | 0.0 | 0.0 |
| Total | 40000.5 | 14400.0 | 44662.0 | 14267.3 | 170.2 | 43036.1 | 11141.1 | 52.6 |

### Example 4

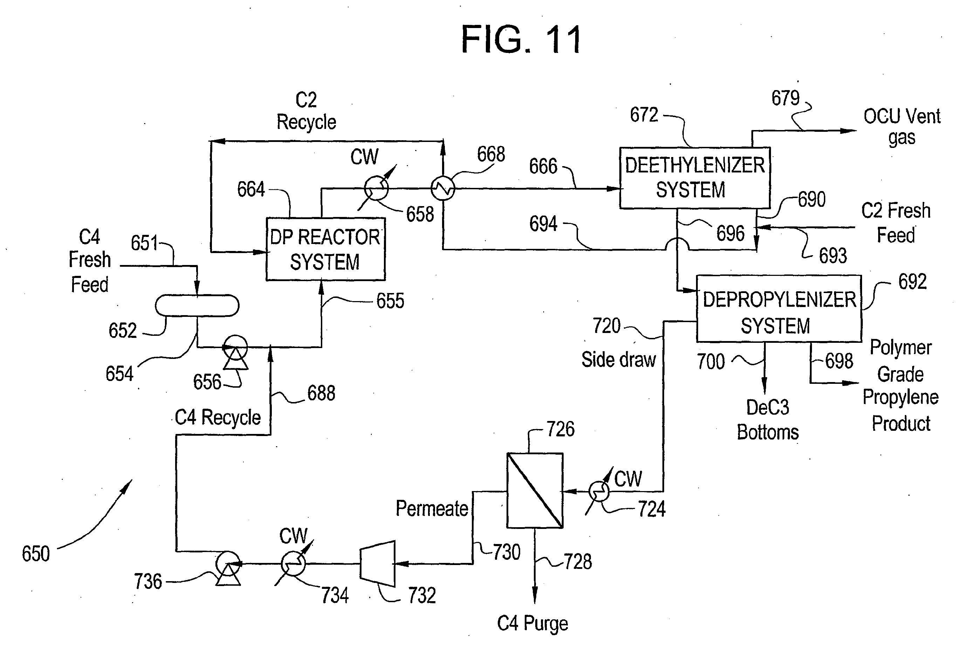

A sophisticated computerized simulation of an optimized metathesis process using the equipment and process flow scheme shown in Fig. 11 was run. The overall process is designated as 650 and is designed to result,in the reaction of 90% of the 1- and 2- butene in the feed. Fresh C4 feed in stream 651 was fed from a feed drum 652 in stream 654 by pump 656, and was combined with C2 recycle from stream 688 in stream 655. Stream 655 was fed to a metathesis reactor system 664. The metathesis product was passed as stream 666 through a heat exchanger 658 where it was cooled, and through another heat exchanger 668, where it was further cooled. The cooled metathesis product stream 666 was then fed to a deethylenizer system 672.

C2s were removed from the top of the deethylenizer system 672 as vent gas stream 679. The C3s and heavier materials from the deethylenizer system 672 were removed in bottoms stream 696. Bottoms stream 696 was fed to a depropylenizer system 692. Recycle stream 690 from the deethylenizer system 672 was combined with fresh C₂ in stream 693 to form stream 694. Stream 694 was heated in heat exchanger 668 and recycled to the metathesis reactor system 664.

In the depropylenizer system 692, polymer grade propylene product was removed in stream 698. The bottoms were removed as stream 700. A side draw stream 720 was removed from the depropylenizer system 692, cooled in heat exchanger 724, and passed through a membrane 726, which preferably is a facilitated transport membrane. The retentate stream 728 was removed as a purge stream. The permeate stream 730 was compressed in a compressor 732, cooled in a heat exchanger 734, conveyed by pump 736, and combined with fresh C₄ feed to form stream 655.

A material balance is shown in Table 8 below and in material balance summary Table 9. The utility consumption and equipment piece count are shown in Table 10 below.

**Table 8: Example 4**

| Component | C4 Fresh Feed | C2 Fresh Feed | C2 Recycle | DeC3 Bottom Purge | PG Propylene Product | C4 Recycle (Permeate) | C4 Purge (Retentate) | OCD Vent Gas |
|---|---|---|---|---|---|---|---|---|
| | Kg/Hr | Kg/Hr | Kg/Hr | Kg/Hr | Kg/Hr | Kg/Hr | Kg/Hr | Kg/Hr |
| Methane | 0.0 | 4.2 | 885.7 | 0.0 | 0.0 | 0.0 | 0.0 | 3.5 |
| Ethylene | 0.0 | 14188.8 | 42252.5 | 0.0 | 0.4 | 0.0 | 0.0 | 49.3 |
| Ethane | 0.0 | 7.1 | 83.3 | 0.0 | 7.1 | 0.0 | 0.0 | 0.1 |
| Propene | 0.0 | 0.0 | 664.9 | 0.0 | 42468.9 | 0.7 | 0.0 | 0.2 |
| Propane | 0.0 | 0.0 | 0.0 | 0.0 | 0.0 | 0.0 | 0.0 | 0.0 |
| i-Butane | 8326.2 | 0.0 | 0.0 | 56.8 | 0.2 | 291.1 | 8269.3 | 0.0 |
| 1-3BD | 0.0 | 0.0 | 0.0 | 0.0 | 0.0 | 0.0 | 0.0 | 0.0 |
| n-Butane | 1826.6 | 0.0 | 0.0 | 17.0 | 0.0 | 63.7 | 1809.6 | 0.0 |
| N- Butenes | 29827.6 | 0.0 | 0.0 | 117.8 | 0.6 | 11836.9 | 1320.3 | 0.0 |
| I-Butenes | 20.1 | 0.0 | 0.0 | 1.2 | 0.1 | 162.6 | 17.0 | 0.0 |
| Heavies | 0.0 | 0.0 | 0.0 | 61.3 | 0.0 | 1667.8 | 0.0 | 0.0 |
| Total | 40000.6 | 14200.1 | 43886.3 | 254.0 | 42477.3 | 14072.7 | 11416.2 | 53.0 |

**Table 9: Overall Material Balances**

| | Comp. Example 4A | Comp. Example 4B | Example 4 |
|---|---|---|---|
| Feeds. MTA | | | |
| MTBE Feed | 267,916 | 320,000 | 320,000 |
| Ethylene | 91,588 | 115.200 | 113,602 |
| Total Feed, MTA | 359,503 | 435,200 | 433,602 |
| | | | |

| Products, MTA | | | |
|---|---|---|---|
| | | | |
| PG Propylene | 272,929 | 344,289 | 339,818 |
| PG Butene 1 | 0 | 0 | 0 |
| PG Hexene 1 | 0 | 0 | 0 |
| | | | |
| OCU Vent Gas | 429 | 421 | 424 |
| | | | |
| | | | |
| | | | |
| Depropylenizer Bottoms | 0 | 1,361 | 2,032 |
| C4 Purge to Cracker | 86,145 | 89,129 | 91,329 |
| | | | |
| C6+ Purge | 0 | 0 | 0 |
| Total Products, MTA | 359,503 | 435,200 | 433,602 |

**Table 10: Utilities and Piece Count**

| | Comp. Example 4A | Comp. Example 4B | Example 4 |
|---|---|---|---|
| PG Propylene Product, MTA | 272,929 | 344,289 | 339,818 |

| Utilities | | | |
|---|---|---|---|
| CW, MMKcal/hr | 8.940 | 12.610 | 8.630 |
| HP Steam, MMKcal/hr | 0.000 | 5.840 | 0.000 |
| LP Steam, MM Kcal/hr | 13.420 | 13.870 | 12.860 |
| Power, MMKcal/hr | 0.119 | 0.118 | 0.439 |
| Fuel, MMKcal/hr | 1.550 | 4.650 | 4.240 |
| Refrigeration @ -27 C | 5.220 | 7.550 | 7.310 |
| Total, MMKcal,hr | 29.249 | 44.638 | 33.479 |
| Utilities per Unit Weight of Propylene Produced, Kcal/Kg | 857 | 1037 | 788 |
| | | | |

| Piece Count | | | |
|---|---|---|---|
| Towers | 2 | 4 | 2 |
| Exchangers | 7 | 13 | 9 |
| Pumps | 8 | 14 | 9 |
| Reactors | 2 | 2 | 2 |
| Heaters | 2 | 2 | 2 |
| Drums | 5 | 10 | 6 |
| Filters | 2 | 2 | 2 |
| Dryers/Treaters | 2 | 2 | 2 |
| Membranes | 0 | 0 | 1 |
| Compressor | 0 | 0 | 1 |
| Total Piece Count | 30 | 49 | 36 |
| | | | |

| Major Equipment Sizing | | | |
|---|---|---|---|
| | | | |

| Deethylenizer | | | |
|---|---|---|---|
| Tower Diameter, m (Top/Bottom | 2.3/2.6 | 2.4/2.6 | 2.3/2.8 |

| Depropylenizer | | | |
|---|---|---|---|
| Metathesis Reactor | | | |
| Length X Diameter, m/m | 3.7X1.8 | 3.7X1.8 | 3.7X1.8 |

| Recycle Pump | | | |
|---|---|---|---|
| Liquid Flow Rate, GPM | 400 | 122 | 128 |

| | | | |
|---|---|---|---|
| (MTA = metric tons per annum) | | | |

The feed rate for Comparative Example 4A was taken to be 267.91 KTA. The C₄ feed rate for Comparative Example 4B and Example 4 was calculated on the basis that the flow rates through the metathesis reactor and to the deethylenizer would be the same as in Comparative Example 4A. This was done to ensure that the major equipment sizes would be the same for comparison purposes. It is noted that due to the improved elimination of inerts, namely n-butane and isobutane, from the recycle loop, in Comparative Example 4 and Comparative Example 4B, a higher fresh C₄ feed read can be tolerated for the same equipment sizes. The recycle pump is smaller in Example 4 and Comparative Example 4B because the inerts are purged out of the loop.

As shown by the above data, more propylene can be made with the same or slightly smaller equipment using the processes of Comparative Example 4B and Example 4, as compared to Comparative Example 4A. This is due to the reduction in recycle rates resulting from the use of an additional separation step. The use of a membrane instead of extractive distillation results a significant reduction in the equipment items hence a lower capital cost. Example 4 uses 26% fewer pieces of equipment to accomplish this advantage than are used in Comparative Example 4B. Comparative Example 4B required 49 pieces of equipment, while the process of Example 4 only required 36 pieces of equipment.

Example 4 has the lowest utility consumption per kilogram of propylene made. Extractive distillation as is used in Comparative Example 4B requires the use of large quantities of energy, due to the condensing and reboiling requirements of the adsorber-stripper system. The use of a membrane instead of extractive distillation results in a 24% reduction in energy consumption per unit weight of propylene produced. While both Comparative Example 4B and Example 4 reduce recycles, only the process of Example 4 reduces both recycles and utilities.

## Claims

1. A process for producing a selected butene, comprising:
(a) obtaining a C4 feed stream comprising C4 paraffins and C4 olefins,
(b) splitting the C4 feed stream to form a first stream comprising a first butene and a second stream comprising a second butene,
(c) isomerizing at least a part of the second stream to convert a portion of the second butene to the first butene, and
(d) recycling at least some of the isomerized part of the second steam to the splitting step (b),
wherein a portion of at least one of the C4 feed stream and the second stream is passed through a facilitated transport membrane to remove butanes, forming at least one purge stream comprising butanes.

2. The process of claim 1, wherein the first butene is 1-butene and the second butene is 2-butene.

3. The process of claim 1, wherein the first butene is a normal butene and the second butene is isobutylene.

4. The process of claim 1, wherein the second butene is converted to the first butene in step (c) by double bond isomerization.

5. The process of claim 1, wherein the second butene is converted to the first butene in step (c) by skeletal isomerization.

6. The process of claim 5, wherein splitting takes place in a catalytic distillation column containing a selective hydrogenation catalyst in which a portion of the 1-butene is converted to 2-butene.

7. The process of claim 1, wherein the C4 feed stream contains poisons, further comprising removing poisons from the C4 feed stream before splitting.

8. The process of claim 1, wherein the C4 feed stream comprises isobutylene, further comprising removing isobutylene from the C4 feed stream before splitting.

9. The process of claim 1, wherein the C4 feed stream contains at least one of acetylenics and dienes, further comprising removing acetylenics and dienes from the C4 feed stream before splitting.

10. The process of claim 1, wherein the recycled portion of the second stream is passed through the facilitated transport membrane prior to isomerization.

11. The process of claim 1, wherein the facilitated transport membrane is promoted with at least one of Cu and Ag.

12. A process for the conversion of C4 olefins, comprising:
(a) obtaining a C4 feed stream comprising C4 paraffins and C4 olefins, including 1-butene and 2-butene,
(b) reacting the C4 feed stream in a metathesis reactor to form a second stream,
(c) fractionating the second stream to form one or more product streams containing ethylene, propylene, pentenes and/or hexenes and a recycle stream primarily containing C4 olefins and C4 paraffins, and
(d) returning the recycle stream to the metathesis reactor,
wherein at least one of the recycle stream and the C4 feed stream is passed through a facilitated transport membrane to remove butanes, forming at least one purge stream comprising butanes.

13. The process of claim 12, wherein ethylene is added to the metathesis reactor and the ethylene reacts with 2-butene to form propylene.

14. The process of claim 12, wherein the feed to the metathesis reactor is primarily 1-butene and the second stream from the reactor contains ethylene and hexene.

15. The process of claim 12, wherein the C4 feed stream contains poisons, further comprising removing poisons from the C4 feed stream before splitting.

16. The process of claim 12, wherein the C4 feed stream comprises isobutylene, further comprising removing isobutylene from the C4 feed stream before splitting.

17. The process of claim 12, wherein the C4 feed stream contains at least one of acetylenics and dienes, further comprising removing acetylenics and dienes from the C4 feed stream before splitting.

18. The process of claim 12, wherein the facilitated transport membrane is promoted with at least one of Cu and Ag.

19. The process of claim 12, further comprising isomerizing the C4 feed stream in an isomerization reactor upstream from the metathesis reactor in order to increase the quantity of 1-butene in the C4 feed stream, and fractionating the isomerized C4 feed stream to form a 1-butene rich C4 feed stream and a 2-butene rich bottoms stream.

20. The process of claim 19, wherein the 2-butene rich bottoms stream is recycled to the isomerization reactor.

21. The process of claim 19, further comprising passing the 2-butene rich bottoms stream through a facilitated transport membrane to remove butanes.

22. The process of claim 12, wherein the one or more product streams include a hexene product stream, further comprising sending the hexene product stream to a hexene isomerization section to maximize 1-hexene production.

23. The process of claim 22, further comprising fractionating the hexene stream to form a 1-hexene stream and a mixed 2- and 3-hexene stream, and recycling the 2- and 3-hexene stream to the hexene isomerization section.

24. The process of claim 19, wherein the one or more product streams include a hexene product stream, further comprising sending the hexene product stream to a hexene isomerization section to maximize 1-hexene production.

25. The process of claim 24, further comprising fractionating the hexene stream to form a 1-hexene stream and a mixed 2- and 3-hexene stream, and recycling the 2- and 3-hexene stream to the hexene isomerization section.

## Patentansprüche

1. Verfahren zum Herstellen eines ausgewählten Butens, umfassend:
(a) Erhalten eines C4-Zufuhrstroms, umfassend C4-Paraffinkohlenwasserstoffe und C4-Olefine,
(b) Splitten des C4-Zufuhrstroms, um einen ersten Strom, umfassend ein erstes Buten, und einen zweiten Strom, umfassend ein zweites Buten, zu bilden,
(c) Isomerisieren wenigstens eines Teils des zweiten Stroms, um einen Teil des zweiten Butens in das erste Buten umzuwandeln, und
(d) Rückführen wenigstens etwas des isomerisierten Teils des zweiten Stroms in den Splittschritt (b),
wobei ein Teil des C4-Zufuhrstroms und/oder des zweiten Stroms durch eine Membran für den erleichterten Transport geleitet wird, um Butane zu entfernen, wodurch wenigstens ein Spülstrom, der Butane umfasst, gebildet wird.

2. Verfahren nach Anspruch 1, wobei das erste Buten 1-Buten ist und das zweite Buten 2-Buten ist.

3. Verfahren nach Anspruch 1, wobei das erste Buten n-Buten ist und das zweite Buten Isobutylen ist.

4. Verfahren nach Anspruch 1, wobei in Schritt (c) das zweite Buten durch Doppelbindungsisomerisierung in das erste Buten umgewandelt wird.

5. Verfahren nach Anspruch 1, wobei in Schritt (c) das zweite Buten durch Skelettisomerisierung in das erste Buten umgewandelt wird.

6. Verfahren nach Anspruch 5, wobei das Splitten in einer einen selektiven Hydrierungskatalysator enthaltenden katalytischen Destillierkolonne durchgeführt wird, in welcher ein Teil des 1-Butens in 2-Buten umgewandelt wird.

7. Verfahren nach Anspruch 1, wobei der C4-Zufuhrstrom Gifte enthält, ferner umfassend das Entfernen der Gifte aus dem C4-Zufuhrstrom vor dem Splitten.

8. Verfahren nach Anspruch 1, wobei der C4-Zufuhrstrom Isobutylen umfasst, ferner umfassend das Entfernen von Isobutylen aus dem C4-Zufuhrstrom vor dem Splitten.

9. Verfahren nach Anspruch 1, wobei der C4-Zufuhrstrom Acetylen-Verbindungen und/oder Diene enthält, ferner umfassend das Entfernen der Acetylen-Verbindungen und Diene aus dem C4-Zufuhrstrom vor dem Splitten.

10. Verfahren nach Anspruch 1, wobei der rückgeführte Teil des zweiten Stroms vor der Isomerisierung durch die Membran für den erleichterten Transport geleitet wird.

11. Verfahren nach Anspruch 1, wobei die Membran für den erleichterten Transport durch Cu und/oder Ag unterstützt wird.

12. Verfahren zur Umwandlung von C4-Olefinen, umfassend:
(a) Erhalten eines C4-Zufuhrstroms, umfassend C4-Paraffinkohlenwasserstoffe und C4-Olefine, einschließlich 1-Buten und 2-Buten,
(b) Reagieren des C4-Zufuhrstroms in einem Metathesereaktor, um einen zweiten Strom zu bilden,
(c) Fraktionieren des zweiten Stroms, um einen oder mehrere Produktströme, enthaltend Ethylen, Propylen, Pentene und/oder Hexene, und einen Rückführstrom, enthaltend vorwiegend C4-Olefine und C4-Paraffinkohlenwasserstoffe, zu bilden, und (d) Zurückführen des Rückführstroms in den Metathesereaktor,
wobei der Rückführstrom und/oder der C4-Zufuhrstrom durch eine Membran für den erleichterten Transport geleitet wird, um Butane zu entfernen, wodurch wenigstens ein Spülstrom, der Butane umfasst, gebildet wird.

13. Verfahren nach Anspruch 12, wobei Ethylen zu dem Metathesereaktor gegeben wird und das Ethylen mit 2-Buten unter Bildung von Propylen reagiert.

14. Verfahren nach Anspruch 12, wobei die Zufuhr zu dem Metathesereaktor vorwiegend 1-Buten ist und der zweite Strom aus dem Reaktor Ethylen und Hexen enthält.

15. Verfahren nach Anspruch 12, wobei der C4-Zufuhrstrom Gifte enthält, ferner umfassend das Entfernen der Gifte aus dem C4-Zufuhrstrom vor dem Splitten.

16. Verfahren nach Anspruch 12, wobei der C4-Zufuhrstrom Isobutylen umfasst, ferner umfassend das Entfernen von Isobutylen aus dem C4-Zufuhrstrom vor dem Splitten.

17. Verfahren nach Anspruch 12, wobei der C4-Zufuhrstrom Acetylen-Verbindungen und/oder Diene enthält, ferner umfassend das Entfernen der Acetylen-Verbindungen und Diene aus dem C4-Zufuhrstrom vor dem Splitten.

18. Verfahren nach Anspruch 12, wobei die Membran für den erleichterten Transport durch Cu und/oder Ag unterstützt wird.

19. Verfahren nach Anspruch 12, ferner umfassend das Isomerisieren des C4-Zufuhrstroms in einem Isomerisierungsreaktor stromaufwärts von dem Metathesereaktor, um die Menge an 1-Buten in dem C4-Zufuhrstrom zu erhöhen, und das Fraktionieren des isomerisierten C4-Zufuhrstroms, um einen 1-Buten-reichen C4-Zufuhrstrom und einen 2-Buten-reichen Sumpfstrom zu bilden.

20. Verfahren nach Anspruch 19, wobei der 2-Buten-reiche Sumpfstrom in den Isomerisierungsreaktor rückgeführt wird.

21. Verfahren nach Anspruch 19, ferner umfassend das Durchleiten des 2-Buten-reichen Sumpfstroms durch eine Membran für den erleichterten Transport, um Butane zu entfernen.

22. Verfahren nach Anspruch 12, wobei der eine oder die mehreren Produktströme einen Hexen-Produktstrom einschließen, ferner umfassend das Zuführen des Hexen-Produktstroms in eine Hexen-Isomerisierungszone, um die 1-Hexen-Produktion zu maximieren.

23. Verfahren nach Anspruch 22, ferner umfassend das Fraktionieren des Hexen-Stroms, um einen 1-Hexen-Strom und einen gemischten 2- und 3-Hexen-Strom zu bilden, und Rückführen des 2- und 3-Hexen-Stroms in die Hexen-Isomerisierungszone.

24. Verfahren nach Anspruch 19, wobei der eine oder die mehreren Produktströme einen Hexen-Produktstrom einschließen, ferner umfassend das Zuführen des Hexen-Produktstroms in eine Hexen-Isomerisierungszone, um die 1-Hexen-Produktion zu maximieren.

25. Verfahren nach Anspruch 24, ferner umfassend das Fraktionieren des Hexen-Stroms, um einen 1-Hexen-Strom und einen gemischten 2- und 3-Hexen-Strom zu bilden, und Rückführen des 2- und 3-Hexen-Stroms in die Hexen-Isomerisierungszone.

## Revendications

1. Procédé pour produire un butène sélectionné, comportant les étapes consistant à :
(a) obtenir un flux d'alimentation en C4 comportant des paraffines en C4 et des oléfines en C4,
(b) séparer le flux d'alimentation en C4 pour former un premier flux comportant un premier butène et un second flux comportant un second butène,
(c) isomériser au moins une partie du second flux pour convertir une partie du second butène en premier butène, et
(d) recycler au moins une partie de la partie isomérisée du second flux à l'étape de séparation (b),
dans lequel une partie d'au moins l'un du flux d'alimentation en C4 et du second flux circule à travers une membrane de transport facilité pour éliminer des butanes, formant au moins un flux de purge comportant des butanes.

2. Procédé selon la revendication 1, dans lequel le premier butène est du but-1-ène et le second butène est du but-2-ène.

3. Procédé selon la revendication 1, dans lequel le premier butène est un butène normal et le second butène est de l'isobutylène.

4. Procédé selon la revendication 1, dans lequel le second butène est converti en premier butène à l'étape (c) par isomérisation de double liaison.

5. Procédé selon la revendication 1, dans lequel le second butène est converti en premier butène à l'étape (c) par isomérisation squelettique.

6. Procédé selon la revendication 5, dans lequel la séparation a lieu dans une colonne de distillation catalytique contenant un catalyseur d'hydrogénation sélectif dans lequel une partie du but-1-ène est convertie en but-2-ène.

7. Procédé selon la revendication 1, dans lequel le flux d'alimentation en C4 contient des poisons, comportant en outre l'élimination des poisons du flux d'alimentation en C4 avant la séparation.

8. Procédé selon la revendication 1, dans lequel le flux d'alimentation en C4 comporte de l'isobutylène, comportant en outre l'élimination de l'isobutylène du flux d'alimentation en C4 avant la séparation.

9. Procédé selon la revendication 1, dans lequel le flux d'alimentation en C4 contient au moins l'un parmi des acétylèniques et des diènes, comportant en outre l'élimination des acétylèniques et des diènes du flux d'alimentation en C4 avant la séparation.

10. Procédé selon la revendication 1, dans lequel la partie recyclée du second flux circule à travers la membrane de transport facilité avant l'isomérisation.

11. Procédé selon la revendication 1, dans lequel la membrane de transport facilité est favorisée avec au moins l'un parmi Cu et Ag.

12. Procédé pour la conversion d'oléfines C4, comportant les étapes consistant à :
(a) obtenir un flux d'alimentation en C4 comportant des paraffines en C4 et des oléfines en C4, incluant du but-1-ène et du but-2-ène,
(b) soumettre à une réaction le flux d'alimentation en C4 dans un réacteur de métathèse afin de former un second flux,
(c) fractionner le second flux afin de former un ou plusieurs flux de produits contenant de l'éthylène, du propylène, du pentène et/ou de l'hexène et un flux de recyclage contenant principalement des oléfines en C4 et des paraffines en C4, et
(d) renvoyer le flux de recyclage dans le réacteur de métathèse,
dans lequel au moins l'un du flux de recyclage et du flux d'alimentation en C4 circule à travers une membrane de transport facilité pour éliminer des butanes, formant au moins un flux de purge comportant des butanes.

13. Procédé selon la revendication 12, dans lequel de l'éthylène est ajouté au réacteur de métathèse et l'éthylène réagit au but-2-ène pour former du propylène.

14. Procédé selon la revendication 12, dans lequel l'alimentation du réacteur de métathèse est principalement du but-1-ène et le second flux du réacteur contient de l'éthylène et de l'hexène.

15. Procédé selon la revendication 12, dans lequel le flux d'alimentation en C4 contient des poisons, comportant en outre l'élimination des poisons du flux d'alimentation en C4 avant la séparation.

16. Procédé selon la revendication 12, dans lequel le flux d'alimentation en C4 comporte de l'isobutylène, comportant en outre l'élimination de l'isobutylène du flux d'alimentation en C4 avant la séparation.

17. Procédé selon la revendication 12, dans lequel le flux d'alimentation en C4 contient au moins l'un parmi des acétylèniques et des diènes, comportant en outre l'élimination des acétylèniques et des diènes du flux d'alimentation en C4 avant la séparation.

18. Procédé selon la revendication 12, dans lequel la membrane de transport facilité est favorisée avec au moins l'un parmi Cu et Ag.

19. Procédé selon la revendication 12, comportant en outre l'isomérisation du flux d'alimentation en C4 dans un réacteur d'isomérisation en amont du réacteur de métathèse afin d'augmenter la quantité de but-1-ène dans le flux d'alimentation en C4, et le fractionnement du flux d'alimentation en C4 isomérisé pour former un flux d'alimentation en C4 riche en but-1-ène et un flux de queue riche en but-2-ène.

20. Procédé selon la revendication 19, dans lequel le flux de queue riche en but-2-ène est recyclé dans le réacteur d'isomérisation.

21. Procédé selon la revendication 19, comportant en outre l'étape consistant à faire circuler le flux de queue riche en but-2-ène à travers une membrane de transport facilité pour éliminer des butanes.

22. Procédé selon la revendication 12, dans lequel le ou les flux de produit incluent un flux de produit hexène, comportant en outre l'envoi du flux de produit hexène à une section d'isomérisation de hexène afin de maximiser la production de hex-1-ène.

23. Procédé selon la revendication 22, comportant en outre le fractionnement du flux de hexène afin de former un flux de hex-1-ène et un flux de hex-2-ène et hex-3-ène mélangés, et le recyclage du flux de hex-2-ène et hex-3-ène dans la section d'isomérisation de hexène.

24. Procédé selon la revendication 19, dans lequel le ou les flux de produit incluent un flux de produit hexène, comportant en outre l'envoi du flux de produit hexène à une section d'isomérisation de hexène afin de maximiser la production de hex-1-ène.

25. Procédé selon la revendication 24, comportant en outre le fractionnement du flux de hexène afin de former un flux de hex-1-ène et un flux de hex-2-ène et hex-3-ène mélangés, et le recyclage du flux de hex-2-ène et hex-3-ène dans la section d'isomérisation de hexène.
